# EUROPEAN PATENT SPECIFICATION

(11) **EP 0 701 624 B1**
(45) Date of publication and mention of the grant of the patent: **27.08.1997**
(21) Application number: 94917572.3
(22) Date of filing: 31.05.1994
(51) Int. Cl.: C12Q 1/10, G01N 33/569, C12Q 1/68

(54) **METHOD FOR THE DETERMINATION OF SALMONELLA**
VERFAHREN ZUR BESTIMMUNG VON SALMONELLA
METHODE DE DETECTION DES SALMONELLES

(30) Priority: 02.06.1993 DK 630/93
(43) Date of publication of application: 20.03.1996
(73) Proprietor: Foss Electric A/S, DK-3400 Hilleroed (DK)
(72) Inventor: GLENSBJERG, Martin, DK-2700 Bronshoj (DK); HANSEN, Flemming, DK-2100 Copenhagen (DK)
(74) Representative: Plougmann, Vingtoft & Partners A/S
(86) International application number: DK9400211
(87) International publication number: WO9428163

(56) References cited:
- EP-A- 0 214 340
- WO-A-88/04326
- WO-A-92/17607
- US-A- 4 563 418
- JOURNAL OF FOOD PROTECTION., vol.49, no.7, July 1986 pages 510 - 514 J. M. DE SMET ET AL. 'Rapid Salmonella detection in foods by motility enrichment on a modified semi-solid Rappaport- Vassiliadis medium.'
- JOURNAL OF FOOD SCIENCE, vol.43, no.5, 1978, CHICAGO US pages 1444 - 1447 B. SWAMINATHAN ET AL. 'Rapid detection of Salmonellae in foods by membrane filter-disc immunoimmobilization technique.'
- Guiraud J. et Galzy P., "L'analyse Microbiologique dans les Industries Alimentaires", Collection Génie Alimentaire, Les EDitions de l'Usine Nouvelle, Paris, 1980, page 68

## Description

### FIELD OF THE INVENTION

The present invention relates to methods for enriching and determining *Salmonella* from a sample, such as a sample of a raw or processed food or feed product.

### GENERAL BACKGROUND

Contamination with bacteria of species belonging to the genus *Salmonella,* in the following referred to as *Salmonella,* of raw and processed foods is a major problem in the food and feed industries. Since the *Salmonella* are ubiquitous, the sources of contamination are numerous with complex routes of transmission.

All *Salmonella* are potentially pathogenic for humans, animals or both. Most serotypes show little host-specificity and may after ingestion cause salmonellosis, a gastro-intestinal disease, in humans.

Although usually mild and self-limiting with recovery within a few days, infections of *Salmonella* may cause severe complications among infants, elderly people and persons with underlying diseases as the infection spreads from the intestinal tract to other systems of the body and may result in serious illness or even death.

Another frequent problem is the immunological responses to the bacterial endotoxins, which may result in hypersensitivity reactions of immediate type (type 1) as well as of delayed type (type IV, cell mediated type) hypersensitivity.

In order to control the contamination of foods in the industry, there is a strong need for fast and reliable means of detecting *Salmonella* in raw as well as processed foods and feeds. The conventional methods for analysing such foods for *Salmonella* conventionally involves the steps of a non-selective enrichment in e.g. buffered peptone water, followed by selective enrichment in broths facilitating the growth of *Salmonella* and inhibiting the growth of other bacteria and subsequent plating on *Salmonella* selective and/or indicative agar-media. Such a process is cumbersome and time-consuming and usually requires 3-5 days to produce a presumptive result.

Over the years, several methodologies have been proposed for an accelerated detection of *Salmonella* in foods. Examples of these include enzyme immunoassays (EIA) (Beumer et al., 1991; Notermans and Wernars, 1991; Emswiler-Rose et al., 1991; D'Aoust and Sewell, 1988b), immunodiffusion (D'Aoust and Sewell, 1988a; Bailey et al., 1991), a latex agglutination assay (D'Aoust et al., 1987), a hydrophobic grid membrane filtration assay (Entis, 1986), a selective motility enrichment technique (Holbrook et al., 1986), and conductance techniques (Smith et al., 1989). Despite the fact that these novel techniques provide more rapid results (42-48 hours) than traditional procedures, they have gained only little acceptance in the food industry. This reflects that there is a need in the industry for methods which further accelerate and simplify the detection of *Salmonella* in foods and feeds.

Guiraud J. and Galzy P., in: "L'analyse microbiologique dans les industries alimentaires", Collection Genie alimentaire, Les Editions de l'Usine Nouvelle, Paris, 1980, page 68, discloses a method for the determination of *Salmonella* which involves an enrichment procedure at 37°C for at least 48 hours in a tetrathionate broth. There are no suggestions of other temperature ranges or durations of the enrichment procedure in tetrathionate broth.

Thus, there is a definite need for a procedure for fast detection of *Salmonella.* Such a procedure should be sensitive, specific, and reliable. For instance, a procedure which is able to detect as little as 1 *Salmonella* per 25 g of sample within 24 hours, the percentage of false positives being less than 10% and the percentage of true positives being at least 80% compared to a standard cultural protocol would be desirable.

### SUMMARY OF THE INVENTION

It has surprisingly been found by the inventors that primary enrichment of a sample of raw food in a buffered *Salmonella*-selective medium at 37°C-43°C for as short a period as 19 hours is an effective means of enriching *Salmonella* present in the initial sample in a way which ensures that an ELISA performed on the enriched samples is highly specific and at least as sensitive as the standard cultural methods for detecting *Salmonella* in samples of raw products.

It has further been found that a primary enrichment of a sample of processed food products (even in a non-selective medium) followed, after a period of time, by an elevation in temperature from about 37°C to 40°C-43°C is an effective means of enriching *Salmonella* present in the initial sample in a way which ensures that an ELISA performed on the enriched samples after 19 hours is highly specific and is at least as sensitive as the standard cultural methods for detecting *Salmonella* in samples of processed products.

The determination performed following the enrichment may be preceded by a selective post-enrichment in order to dilute food-components which might possibly interfere non-specifically in the subsequent analyses. To compensate for the dilution of *Salmonella,* the post-enrichment is normally performed for a period of time in the range of about 2 to about 6 hours at a temperature in the range of about 40°C-43°C. More specifically, the duration of the post-enrichment is advantageously in the range of about 2.0-4.5 hours, preferably in the range of 2.5-3.5 hours, and most preferably the duration of the post-enrichment is about 3 hours. Whether such a selective post-enrichment should be performed mainly depends on the characteristics of the assay employed in the determination step: In case the assay is substantially insensitive to otherwise interfering or even cross-reacting substances in the assayed sample, there is no need for such a post-enrichment.

The invention is based on the above-mentioned and other findings and utilizes a strategy where *Salmonella* is subjected to a selective enrichment as soon as the cells are ready therefor. The main philosophy behind this is to inhibit competing microflora, so that overgrowth of *Salmonella* by the microflora is counteracted, also with respect to the capability of competing microflora to be detected in the determination. Even though counteracting the overgrowth of *Salmonella* by the strategy according to the present invention will also to some extent apply a selection pressure on *Salmonella,* the balanced selectivity utilized according to the invention is nevertheless in great favour of *Salmonella* with respect to obtaining a successful detection of any *Salmonella* in a sample, such as will appear from the following description and documentation.

Expressed in another manner, the present invention is primarily based on the idea of adapting the enrichment procedure to the determination procedure, rather than adapting the determination procedure to a classical enrichment procedure, the latter being the conventional approach. Particular features of the invention are based on the discoveries that
by combining the selective substance tetrathionate with an elevated incubation temperature, a particularly high sensitivity and specificity with regard to detection of *Salmonella* is obtained, especially in raw products, and
by combining the use of the antibiotic agent novobiocin with an elevated incubation temperature, correspondingly excellent results are obtained, especially in processed products.

In summary, the invention provides novel methods which permit the detection of *Salmonella* within 24 hours with a very high degree of sensitivity and specificity.

### DETAILED DISCLOSURE OF THE INVENTION

In one aspect, the invention relates to a method for the determination of bacteria belonging to the genus *Salmonella* in a sample, in particular a raw sample, the method comprising one of the three enrichment procedures 1)-3) explained below, and a determination to identify and/or quantify *Salmonella* present in the enrichment medium after the proliferation, the determination comprising an assay step which is based on the identification and/or quantification of a target molecule or a molecular interaction involving a target molecule and which is different from traditional agar plating techniques as defined herein. Examples of assays based on the identification and/or quantification of a target molecule or a molecular interaction involving a target molecule are: assays involving an immunological reaction, such as an immunoassay, e.g. a Radio Immune Assay (RIA), an Enzyme Immune Assay (EIA), an Enzyme Linked Immune Sorbent Assay (ELISA), a Fluorescence Antibody technique (FA), an immunoassay comprising the use of liposomes, micelles or liposome-like particles in the determination phase, an assay which involves immune immobilisation, an assay which involves immune agglutination such as latex agglutination; assays wherein the determination step comprises a DNA/DNA-hybridisation assay, a RNA/RNA-hybridisation assay or a DNA/RNA-hybridisation assay; assays wherein the determination step comprises a polymerase chain reaction (PCR) or similar *in vitro* nucleic acid multiplication steps; and assays wherein the determination step comprises the use of peptide nucleic acids (PNA; cf. Nielsen P E *et al.,* 1991).

According to the invention it is an advantage to limit the duration of the assay step. Normally the assay should have a duration of at the most 7 hours, but shorter durations are preferred, such as at the most 6, 5, 4, 3, 2, and 1 hours.

Other interesting types of determination steps which may be a part of the method of the invention are listed in the following:
determination steps comprising the use of lectines or similar assays involving receptor binding;
determination steps comprising the use of bacteriophages (of which the bacteriophage Felix 01 is an especially interesting possibility) ;
determination steps comprising a metabolic conversion by the *Salmonella* of a substance which results in a change in the electric resistance or impedance of the medium containing the *Salmonella* and wherein the detection of the *Salmonella* is accomplished by measuring this change;
determination steps comprising an assay for the ability of *Salmonella* to migrate on or through an agar matrix;
determination steps comprising an assay involving a filtration step, wherein the bacteria are separated from the medium and are retained on a filter (here, an assay involving a hydrophobic grid membrane filter method (HGMF) is especially interesting);
determination steps comprising an assay involving a radiometric method; and
determination steps comprising an assay involving a chromatographic separation.

Specific embodiments of such assays are to a large degree described in the references listed on page 2 in this specification.

A large number of such assays are known in the art. In the examples section, the use of an ELISA assay is described, but it will be understood that such a choice is of limited importance in carrying out the invention. For instance, assays involving hybridization between nucleic acids are also very interesting candidates, as such assays can be tuned to very high degrees of species specificity as well as of strain specificity. Further, such assays may be less sensitive to interference from other bacteria or substances, thus reducing the need for a selective post-enrichment and thereby simplifying the methods of the invention.

It will be understood that the above-mentioned assays form part of the "determination step" by which is herein meant the phase of the method wherein the determination of *Salmonella* takes place.

By the term "determination" is meant the quantitative or qualitative assessment of the presence of *Salmonella* in a sample. Frequently, a qualitative assessment is the goal of the method according to the invention, as the important thing often is to establish merely the presence or absence of *Salmonella.* However, in situations where either a certain limited contamination is tolerated or a quantitative assessment in itself is interesting (e.g. when assessing the level of *Salmonella* in a sample from an animal or a human being) the determination may be quantitative. In the latter case it is usually necessary to compare with an external standard containing a known amount of *Salmonella,* the standard being treated in a manner parallel to that of the sample.

The assay employed may be any convenient assay for determining the presence of *Salmonella.* However, the assay is not a "traditional agar plating technique" as used in many standard protocols for the detection of *Salmonella,* for instance the plating technique described in ISO 6579.

An assay may be subject to interference resulting from non-specific interactions between substances contained in the original sample and components of the assay. Such interference may give rise to false-positive results as well as false-negative results.

It will be understood that the problems caused by such interfering substances stem from the fact that the initial concentration of non-microbiological material in the primary enrichment medium is high and will remain so throughout the primary enrichment, whereas the initial density of microorganisms (both *Salmonella* and others) is normally rather low. Thus, an enrichment procedure according to the invention will have no effect on the inherent interference of substances already present, whereas the enrichment procedure according to the invention will eliminate or greatly reduce interference from cross-reacting microorganisms.

When used herein, the term "cross-reactivity" refers to the characteristic ability of *non-Salmonella* bacteria or molecules originating from non-Salmonella bacteria to elicit a false-positive result or signal. Accordingly a "cross reacting microorganism" exhibits cross-reactivity or "cross-reacts". It will be understood that cross-reactivity is a result of the presence of a common feature of the cross-reacting substance/microorganism and the *Salmonella* assayed for. Cross-reactivity should therefore be distinguished from non-specific phenomena such as non-specific interactions between substances and components of an assay.

Apart from an assay, the determination step may therefore comprise a "post-enrichment", by which term is meant a selective enrichment performed by transferring material from the primary enrichment to a volume of a selective enrichment medium so as to dilute substances in the primary enrichment medium which might otherwise interfere in the assay. As mentioned above, the necessity for a post-enrichment is highly dependent on the type of assay employed and it is mainly when employing assays which are subject to the above-mentioned non-specific interactions that a post-enrichment will prove valuable.

In this context the term "signal" refers to the measurable output of an assay testing for the presence of a chosen characteristic of *Salmonella.* Said characteristic may be any biological or chemical feature which distinguishes *Salmonella* from other microorganisms in the sample assayed for the presence of *Salmonella,* such as DNA-fragments, RNA-fragments, antigenic determinants, enzymes, receptors and other macromolecules.

In the present context the term "cut-off value" refers to the minimal signal from an assay which is regarded as a positive signal. In the experiments performed utilising the ELISA-2 technique, this cut-off value is defined as two times the signal obtained when assaying a substantially *Salmonella-free* sample consisting of WASH, whereas the cut-off value in the experiments utilising the ELISA-1 technique is defined as a percentage of a well-defined standard sample of *Salmonella* (for details, cf. the example section).

Accordingly, in this context the term "positive signal", *i.e*. a final or presumptive result which states that the sample contains *Salmonella,* denotes a signal above a chosen cut-off value and the term "negative signal", *i.e.* a final or presumptive result which states that the sample does not contain *Salmonella,* denotes a signal below the cut-off value.

A "true-positive" signal or result is herein defined as a positive signal or result which can be confirmed when culturing from the medium of the primary enrichment which resulted in the positive signal. The culturing might be performed as described in example 5 herein.

A "false-positive" signal or result is defined herein as a positive signal or result which cannot be confirmed when culturing from the medium of the primary enrichment which resulted in the positive signal in the determination. The culturing may e.g. be performed as described in example 5 herein. Correspondingly, a "false-negative" signal or result is defined as a negative signal or result which cannot be confirmed as negative when culturing from the medium of the primary enrichment which resulted in the negative signal in the determination.

The term "sample" as used herein, defines any specimen taken from any possible source which is suspected of harbouring *Salmonella.* Thus, samples to be subjected to the method according to the invention may originate from sources such as raw and processed products (as defined below), but also from sources such as faeces, gastric juice, urine, blood, lymph, cerebrospinal fluid, biopsies, and other samples taken from animals, including human beings. Another important group of samples are those of environmental origin: dust, swabs, fluff etc.

By the term "product" is meant all kinds of samples which are investigated for the presence of *Salmonella,* whereas the term "processed product" is defined as a product, for instance a food or a feed, which has been exposed to any kind of processing (except freezing) that may lead to sublethal damage of *Salmonella* (e.g. heating, drying, curing, acidification) and which typically contains less than 5% by weight raw material as defined below. The term "raw product" or raw material is thus a product which has not been subjected to the above-mentioned processing. A raw product typically contains
- at least 5% by weight raw meat and/or fish, and/or
- at least 5% by weight of raw egg, and/or
- at least 5% by weight of raw milk, and/or
- at least 5% by weight of raw vegetables and/or fruit, and/or
- at least 5% by weight of a environmental sample selected, e.g., from sewage, and faeces.

Enrichment procedure 1) will be discussed first. This enrichment procedure comprises transferring the sample to an aqueous growth medium for *Salmonella,* the growth medium being buffered to a pH between 5.6 and 9.3, tetrathionate and/or another selective substance being added to or generated in the medium before, simultaneously with or after the transfer, and keeping the resulting mixture at a temperature of at the most 38°C for a period of time of between 19 min, and 8 hours after the transfer of the sample (pre-enrichment period) and then increasing the temperature of the mixture to 39-43°C and keeping the mixture at the increased temperature of 39-43°C for a period of time after the increase of the temperature (enrichment period), the sum of the periods of time of the pre-enrichment period and the enrichment period being between 10-48 hours. Enrichment procedure 1) is characterized by utilisation of a selective substance, in particular tetrathionate.

The growth medium used in the enrichment procedure above and in the other enrichment procedures according to the present invention is a medium containing basal assimilable nutrient sources, micronutrients and essential growth factors. The medium should have an osmotic pressure adapted for cultivation of *Salmonella* and should be buffered to a pH in the range of 5.6 to 9.3. It is preferred that the medium is free from or substantially free from sugars, or, if the medium does contain sugars, that their concentration is so adapted to the buffer capacity of the medium that the pH of the medium will not be brought outside the above-mentioned range due to acid formation as a result of fermentation of the sugars, at least within the first 10-12 hours. An excellent medium fulfilling these criteria is buffered peptone water (BPW).

In the present context, the term "enrichment" simply designates cultivation in an aqueous growth medium for *Salmonella* which facilitates the proliferation of *Salmonella,* whereas "pre-enrichment" designates a preceding cultivation at a temperature which is lower than the temperatures during the subsequent enrichment. A "primary enrichment" is substantially the first cultivation in an aqueous growth medium for *Salmonella* which takes place within the first 15-30 hours.

The main strategy of enrichment procedure 1) is to apply a selective substance at an early stage of the procedure, thereby exposing non-*Salmonella* to inhibiting conditions as soon as possible, at the same time adapting the concentration of the selective substance and other conditions, including the temperature, so that only little inhibition of *Salmonella* will occur, and then, when *Salmonella* has adapted to the conditions, making the selective conditions more stringent by increasing the temperature, whereby *Salmonella* will show a good growth rate compared to many non-*Salmonella* and, in addition, the non-*Salmonella* will tend to produce less false positive due to less development of e.g. flagella which might interfere in e.g. a later ELISA determination. While the pre-enrichment period may have a length in the range 10 minutes to 8 hours, it is normally in the range from ½ hour to 7 hours and often in the range from 1 hours to 6½ hours, such as in the range from 2 hours to 6 hours, in particular from 3 hours to 5 hours. A preferred length of the pre-enrichment period is about 4 hours.

In the above enrichment procedure (and in the other enrichment procedure described herein) it is possible to generate the selective substance in the growth medium at a certain point after the enrichment has commenced. Such a generation of selective substance is according to the invention preferably substantially instantaneous, but may be a gradient-like process, wherein the concentration of the selective substance slowly increases. When the selective substance is tetrathionate, the generation of tetrathionate is performed by keeping thiosulphate in the medium and later adding iodine, thereby obtaining tetrathionate in the medium.

"Selective principles" when used herein, are means of facilitating the growth of *Salmonella* relative to the growth of most other microorganisms (non-*Salmonella*) in an aqueous growth medium, *i.e.* a selective principle can be a substance (selective substance) which, when added to a *Salmonella* growth medium in a sufficient amount, inhibits the growth of *Salmonella* to a substantially lesser degree than most other microorganisms or a selective principle can be adjustment of a physical parameter, e.g. temperature, to a value which inhibits the growth of *Salmonella* to a substantially lesser degree than most other microorganisms. A selective principle further denotes a means which ensures that a chosen assay for detecting the presence of *Salmonella* performed after an enrichment which makes use of a selective principle will substantially not be influenced by the presence in the initial sample of otherwise cross-reacting microorganisms or molecules.

Hence, "selective enrichment" denotes an enrichment procedure which makes use of a selective principle and consequently a "non-selective enrichment" is a enrichment procedure which does not make use of selective principles.

It will be appreciated that other microorganisms might very well be present in the assayed sample and even in higher concentrations than the *Salmonella* tested for, but that these microorganisms do not interfere with the chosen assay, because their cross-reactivity is depressed as a consequence of the enrichment procedure.

By a "primary selective enrichment" is meant a primary enrichment which is turned into a selective enrichment within the first 8 hours of the primary enrichment.

In accordance with what is explained above, the terms "enrichment broth", "enrichment medium", "primary enrichment broth", "primary enrichment medium", "selective enrichment broth", "selective enrichment medium", "primary selective enrichment broth" and "primary selective enrichment medium" denote broths or media used for the corresponding enrichments.

It will be understood that a primary selective enrichment has as its main objective to provide sufficient amounts of *Salmonella* in order to perform a determination for the presence of *Salmonella* which will result in a positive signal in an assay if any *Salmonella* were present in the initial sample. An enrichment procedure should therefore be evaluated with respect to its ability to enrich an initial small concentration of *Salmonella* into a final sufficiently high concentration of *Salmonella* in order to perform a successful assay. Therefore, the sensitivity of a combined method comprising an enrichment procedure and a determination for *Salmonella* is dependent of the efficiency of the enrichment as well as the sensitivity of the determination employed.

The term "sensitivity" as used herein, is defined as the ability of a method to detect the *Salmonella,* if present, in the initial sample. If it is stated that a method has a better sensitivity than a standard protocol it means that the method statistically will find more true positive results than the standard protocol when testing a large number of random samples.

The duration of the enrichment period is normally adapted so that the sum of the pre-enrichment period and the enrichment period is in the range of 10-48 hours, preferably 15-30 hours, in particular 17-24 hours, whereby any *Salmonella* in the sample will normally be multiplied to an acceptable level while, at the same time, the total duration of the enrichment procedure is satisfactorily short. It will by understood that wherever pre-enrichment and enrichment periods are discussed herein, the periods stated will be the total time under the conditions in question; although it is normally not preferred, the pre-enrichment or enrichment period may be interrupted, e.g. when adding or generating a selective substance or for other reasons.

Thus, the combination of a suitable concentration of tetrathionate and a temperature in the range of 39-43°C makes it possible to obtain on the one hand an excellent growth of *Salmonella* and on the other hand a very substantial suppression of cross-reactions from non-*Salmonella* which would lead to false positives. While the concentration of tetrathionate in the mixture may, in principle, be in the range of about 5-40 mM or even outside that range, the concentration of tetrathionate will normally be in the range of about 7-35 mM, usually in the range of about 9-30 mM and preferably in the range of about 11-28 mM, such as in the range of about 13-26 mM, e.g. about 14-25 mM, preferably in the range of about 15-23 mM and in many cases preferably in the range of about 16-22 mM, in particular in the range of about 17-21 mM such as about 18-20 mM. The most preferred concentration of tetrathionate is about 19 mM. As indicated above, the specific concentration to be selected in particular situation will especially depend on the temperature and the presence of any other selective substances. As an interesting supplement to tetrathionate, novobiocin can be added in order to suppress *Proteus* and many Gram-positive bacteria. The optimum amount of Novobiocin is about 2.5 µg/ml. As a guideline, the preferred temperature within the range of 39-43°C will normally be between 40 and 42°C during at least a substantial part of the enrichment period, in particular in the range between 40.5 and 41.5°C, most preferably 41°C.

While the medium is buffered to a pH between 5.6 and 9.3 at the beginning of the enrichment procedure, it cannot be ruled out that in special cases, e.g. when working with samples of potentially acidic pH such as raw milk products, the pH will not remain in this range during the whole enrichment procedure, although it is generally preferred that there is sufficient buffer capacity present to keep pH in the range. Within the range stated, it is preferred to keep pH in the subrange between 6.7 and 7.3, in particular just about 7.0.

Preferably, the tetrathionate or other selective substance, such as novobiocin, is present in the medium prior to the addition of the sample to the medium. In the case of tetrathionate, it is to be noted that this substance is normally produced *in situ* by addition of iodine to the medium whereby the iodine will react with thiosulphate which has been added to the medium in advance. However, it will be understood that although it is normally not preferred, tetrathionate and/or another selective substance may be added to or generated in the medium at a time after the addition of the sample to the medium. In such case, the addition or generation of the selective substance will normally take place at the most 4 hours after the transfer, suitably at the most 2 hours after the transfer, preferably at the most 1 hour after the transfer or most preferably at the most 10 minutes after the transfer.

While selenite, such as sodium hydrogen selenite (in the following termed selenite), is a normally used selective substance in *Salmonella* enrichment, including the standard protocol, e.g., the AOAC, selenite is not a preferred substance in the present context, in particular because it tends to incur problems with false positives. For this reason, it is preferred that no selenite is present in the enrichment medium, or that any presence of selenite is limited to a concentration which corresponds to the selenite concentration in a solution containing at the most 0.5% w/v sodium hydrogen selenite.

An important feature of a combined primary selective enrichment and subsequent determination for the presence of *Salmonella* is the specificity; it will be understood that such a combined method might give rise to positive results which cannot be attributed to the presence of *Salmonella.* Therefore, a method like the methods according to the invention should also be evaluated with respect to its ability to avoid false-positive results. Thus, the conditions of the enrichment procedure are adapted to the determination so that a high quality determination is obtained.

When used herein, the term "specificity" refers to the ability of a method to avoid producing false-positive results or signals. It will be understood that a method producing a low number of false-positive results or signals is a method with a high degree of specificity.

In order to assist the adaption, a set of simple and reproducible tests has been developed. Two of these tests employ readily available standard pure cultures which are available from national collections, such as ATCC:

Test 1) is a test for the ability of the method to detect *Salmonella* and is performed by performing the above-discussed enrichment and determination procedures wherein the selective enrichment medium has been inoculated with a strain of *Salmonella* to a starting concentration of about 15 cells/ml and 10% randomly selected minced raw meat has been added to the enrichment medium at the beginning of the enrichment period and the resulting mixture has been maintained at the temperature or the temperatures for the enrichment period, and the test is considered as passed if it will produce, in the determination, a positive result in at least 90% of all cases when testing a panel of randomly chosen *Salmonella* serotypes.

Test 2) is a test for the ability of the method to avoid false positives and is performed by performing the above-discussed enrichment procedure and the determination wherein the selective enrichment medium has been inoculated at the beginning of the enrichment period with a strain of one of the non-*Salmonella* species
*Citrobacter freundii,*
*Escherichia coli,* and
*Enterobacter cloacae,*
to a starting concentration thereof of about 100 cells/ml, the selective enrichment medium being substantially free from microorganisms other than the non-*Salmonella* strain inoculated, and the resulting mixture has been maintained at the temperature or the temperatures for the enrichment period, and the test is considered passed when the percentage of false positives in the assay is at the most 15% within each species when testing a panel of 20 strains randomly selected from the non-*Salmonella* species.

It will be understood that if a particular setup will not pass one or both of the tests, the person skilled in the art will understand how to fine-tune the conditions of the enrichment to the conditions of the determination, e.g. by increasing the enrichment temperature and/or the concentration of the selective substance and/or decreasing the pre-enrichment time in case test (2) gives rise to too many false positives, and by, e.g., reducing the stringency of the selective principles in order to avoid false negatives in test (1).

While a minimum of true positives in test 1 is 90%, it is preferred that this number is higher, such as 92% or 95% or better 97% or even up to 99.5% or better 99.9% in test 1.

In addition, test 2 should result in no more than 15% of false positives, preferably 12% or 10% or more preferably 7% or 5% and even down to 1% or ½%.

A more ultimative test comprises a calibration of the method and the setup against the standard protocol NMKL no. 71 (Nordic Committee on Food Analysis method no. 71, 1991, fourth edition), with the exceptions that RVS as defined herein is used instead of RV as defined herein and RVS is incubated at 42°C and that XLD as defined herein is also used as a plating medium, in such a manner that the determination results in at least 80% true positive results as defined herein compared to the standard protocol, and gives rise to at the most 10% of false positive results as defined herein when testing 100 samples of minced meat (test 3), and the weight of the portion of sample to be tested by each of the two methods being 25 g, the 100 samples being provided from 10 different retails, each of which delivers 10 different samples, 20 of the samples provided having been inoculated with about 15 *Salmonella typhimurium* cells per sample and 20 of the samples provided having been inoculated with about 15 *Salmonella enterititis* cells per sample.

In such a calibration, the minimum requirement may be 80% true positives by the method of the invention compared to the standard protocol, but the goal will often be higher such as 85%, 90%, 92%, 95%, 97%, 98%, 99%, and even 100% or even more true positives. With regard to the number of false positives, the maximum acceptable level will normally be 10%, but the goal will be lower percentages such as 8%, 5%, 3%, 2%, and 1% or even 0%.

One of the advantages of the method of the invention is that it reduces the total detection time, compared with the standard protocol. Thus, the sum of the enrichment procedure and the determination step will normally be at the most 56 hours (such as at most 48 hours, 40 hours, 34 hours, 32 hours, 28 hours), but more realistic embodiments will normally produce results within 24 hours or even within 22 or 20 hours.

The method described above is preferably used when determining *Salmonella* in samples which are different from processed products, i.e raw products as defined above.

Enrichment procedure 2) will now be described. This procedure involves transferring the sample to an aqueous growth medium for *Salmonella,* the growth medium being buffered to a pH between 5.6 and 9.3, the growth medium with the sample being kept at a temperature of 39-42.5°C, tetrathionate and/or another selective substance different from selenite being added to or generated in the medium before, simultaneously with or at the most 8 hours after the transfer, and keeping the resulting mixture at a temperature of 39-42.5°C for a period of time of between 10-48 hours from the transfer (enrichment period).

The strategy of this enrichment procedure is to use the temperature as the main selective parameter right from the start of the enrichment procedure, tetrathionate and/or another selective substance being applied in adapted amounts preferably as soon as practically possible. With respect to details concerning this strategy, its coordination with the determination and tests 1)-3), reference is made to the above discussion of enrichment procedure 1) and to the claims. However, an important variant in enrichment procedure 2) is where novobiocin is added to the medium as the only selective substance before, simultaneously with or after the transfer of the sample, the amount of novobiocin in this case being in the range of about 1 to 100 µg per ml, in particular 1-20 µg per ml, especially 2-13 µg per ml. In this case, the enrichment temperature is preferably in the range of 40-42°C.

Finally, enrichment procedure 3) comprises transferring the sample to an aqueous growth medium for *Salmonella,* the growth medium being buffered to a pH between 5.6 and 9.3, tetrathionate being added to or generated in the medium before, simultaneously with or at the most 3 hours after the transfer, and keeping the resulting mixture at a temperature of about 36.0-39.0°C for a period of time of between 15-30 hours after the transfer of the sample (enrichment period). Also with respect to this procedure, the discussion given above concerning procedure 1) is relevant to the extent appropriate, with the important exceptions that procedure 3) gives the best results when keeping the temperature in the range of 36-39°C and that tetrathionate should be added or generated within about 1 hour, preferably within 5 minutes, from the addition of the sample. When using enrichment procedure 3, the assay step should have a duration of at most 7 hours, and the sum of the time of the enrichment and the determination should be at most 40 hours.

While all of the above variants are predominantly suitable in connection with raw samples, another major aspect of the invention relates to a method for enriching and determining *Salmonella* in particular in processed products. This aspect, which will be discussed in the following, comprises performing an enrichment procedure which comprises either
a) transferring the sample to an aqueous growth medium for *Salmonella,* the growth medium being buffered to a pH between 5.6 and 9.3 and containing substantially no bacterial growth-inhibiting substance, and keeping the resulting mixture at a temperature of at most 39.0°C for a period of time of at least 1 minute (pre-enrichment period), and then
   increasing the temperature of the mixture to 39.5-43°C and optionally adding novobiocin and/or another selective substance
   and keeping the mixture at the increased temperature of 39.5-43°C for a period of time from the temperature increase (selective enrichment period), or
b) transferring the sample to an aqueous growth medium for *Salmonella,* the growth medium being buffered to a pH between 5.6 and 9.3, novobiocin and/or another selective substance being added to the medium before, simultaneously with or after the transfer, and keeping the resulting mixture at a temperature of at most 39.0°C for a period of time of at least ½ hour from the transfer or the addition of novobiocin or another selective substance, whichever the later (pre-enrichment period), and then
   increasing the temperature of the mixture to 39.5-43°C and optionally adding novobiocin or another selective substance
   and keeping the mixture at the increased temperature of 39.5-43°C for a period of time from the temperature increase (selective enrichment period), or
c) transferring the sample to an aqueous growth medium for *Salmonella,* the growth medium being buffered to a pH between 5.6 and 9.3, novobiocin being added to the medium before, simultaneously with or after the transfer, and keeping the resulting mixture at a temperature of at most 39.0°C for a period of time of between 15-30 hours from the transfer or the addition of novobiocin or another selective substance, whichever the later (enrichment period),
   and then performing a determination to identify and/or quantify *Salmonella* present in the enrichment medium after the proliferation.

When using enrichment procedure C, the assay step should have a duration of at most 7 hours, and the sum of the time of the enrichment and the determination should be at most 40 hours.

The strategy behind this aspect of the invention is, in principle, the same as the strategy behind the first-mentioned aspect, that is, that the sample should be subjected to selective principles as soon as *Salmonella* is ready therefor, but the present aspect takes into special considerations that *Salmonella* in a processed sample is sublethally damaged and must be resuscitated appropriately before it can be subjected to any pronounced selectivity.

The enrichment procedures according to this aspect of the invention are believed to be unique and advantageous, not only in connection with an assay determination (as defined above) to which they are perfectly adaptable, but also quite generally as enrichment procedures in their own right which can be used in combination with the conventional selective enrichment media (such as RV and TTB as defined below) as well as with conventional agar plating. However, for most purposes, the determination preferably comprises an assay step which is based on the identification and/or quantification of a target molecule or a molecular interaction involving a target molecule, and which assay step is different from agar plating as defined herein, confer the discussion in connection with the aspect especially relating to raw products.

The conditions of each of the enrichment procedures are preferably so adapted to the determination that
- a test (test 4) comprising the enrichment procedure and the determination wherein the aqueous growth medium has been inoculated with heat-treated cells of a strain of *Salmonella* to a starting concentration of about 1000 heat-treated cells/ml and 5% non-fat dry milk has been added to the medium at the beginning of the enrichment procedure will produce, in the determination, a positive result in 90% of all cases when testing a panel of randomly chosen *Salmonella* serotypes, and
- a test (test 5) comprising the enrichment procedure and the determination, wherein the aqueous growth medium has been inoculated with a strain of one of the *non-Salmonella* species
   *Citrobacter freundii,*
   *Escherichia coli,* and
   *Enterobacter cloacae,*
   to a starting concentration thereof of about 100 cells/ml at the beginning of the enrichment procedure will give rise to a percentage of false positives in the determination of at the most 25% within each species of a panel of 20 randomly selected species of the of the non-*Salmonella* strains.

The heat treatment of the cells mentioned above should be performed in water at 51°C containing 0.85% NaCl for 10 minutes.

Also in this aspect, a calibration against a standard cultural protocol, NMKL no. 71 (Nordic Committee on Food Analysis method no. 71, 1991, fourth edition), is performed in such a manner that the determination results in at least 80% true positive results compared to the standard protocol, and gives rise to at the most 10% of false-positive results as defined herein when testing (test 6) a total of 50 samples of 50 g of milk powder randomly chosen with respect to batch and type, each sample being divided in a set of two parts of 25 g, one part of each set being subjected as sample to a method according to the invention for enriching and determining *Salmonella* in particular in processed products and the other part of each set being subjected as sample to the standard cultural protocol, 25 of the 50 sets of 25 g parts being spiked in the following manner:
- one 25 g part of the set, subjected to the standard cultural protocol, is supplemented by adding, at the beginning of the enrichment procedure of the protocol, 0.1-1 g non-fat dry milk containing about 5 CFU *Salmonella panama* or *Salmonella typhimurium* to the aqueous growth medium used in the protocol,
- the other 25 g part of the set, subjected to a method according to the invention for enriching and determining *Salmonella* in particular in processed products, is supplemented by adding, at the beginning of the enrichment procedure, 0.1-1 g non-fat dry milk containing about 5 CFU *Salmonella panama* or *Salmonella typhimurium* to the aqueous growth medium.
"CFU" (Colony forming units) is determined using a conventional dilution and plating technique.

With respect to the results to be aimed at in tests 4)-6), the same considerations apply as are discussed above in connection with the aspect in particular relating to raw products, with the exception that test 5 is generally somewhat less stringent than the corresponding test 2 discussed above, such as will appear from the relevant claims.

In variant a) or variant b), the pre-enrichment period is normally at least ½ hour, such as 1-10 hours, preferably 1-8 hours, especially 2-6 hours, such as 3-5 hours and most preferably 3½-4½ hours. An especially preferred duration of the pre-enrichment period is about 4 hours.

With respect to the total enrichment period, the duration of the determination step and the character of the assays, the same considerations apply as are discussed above.

In all of the above aspects, the sample will be prepared according to the regulations which apply in any particular country to the particular field of product to which the sample pertains. Thus, sample preparation may comprise grinding, comminution, homogenisation, etc., in accordance with the well-known standard methods.

### EXAMPLES

### Materials and methods

As *Salmonella* must be determined in concentrations as low as one bacterium in 25 g of food it is necessary to perform an enrichment of the sample prior to the ELISA analysis.

The enrichment procedure used in the examples consists of a selective primary enrichment (15-30 hours) in accordance with the principles described above, and a post-enrichment in modified M-broth in some of the examples as a part of the determination step in order to dilute food components which might possibly interfere in the subsequent analyses. To compensate for the dilution of *Salmonella* the post enrichment is normally performed for a period of time in the range of about 2 to about 6 hours at a temperature in the temperature range from 40°C-43°C. The modification of the M-broth consists of adding either tetrathionate or novobiocin.

Thus, the total enrichment procedure can be performed in a total of about 15 to about 36 hours.

The following media were used in the experiments described in the examples below:
- API-20E system:: Bio Mérieux 20100.
- BGA:: Brilliantgreen agar (Merck 7273/Oxoid CM 329).
- BPW:: Buffered Peptone Water (Gibco 152-03812/Oxoid CM501).
- conjugate 1:: *Salmonella* monoclonal antibody conjugated to the enzyme β-galactosidase (2 µg/ml).
- conjugate 2:: *Salmonella* polyclonal antibody conjugated to biotin.
- AV-GAL:: Streptavidin conjugated to β-galactosidase (Zymed) diluted 2000 times in 1% bovine serum albumin.
- H-serum:: Polyvalent H-serum (Difco 2406).
- LIA:: Lysine Iron Agar (Difco 0849).
- MB:: M-Broth (Difco 0940).
- MBN:: M-Broth supplemented with novobiocin (Sigma N 1628, 10 µg/ml).
- O-serum:: Polyvalent O-serum (Difco 2264).
- ONPG broth:: O-Nitrophenyl-β-D-galactopyranoside (Sigma N 1127) prepared as described by Cowan, 1974.
- particle sol. 2.:: Immunospheres (Dynabead® (a registered trademark owned by Dynal, Norway), 2.0 mg/ml) coated with monoclonal antibodies directed against a *Salmonella* common flagellar antigen.
- particle sol. 1.:: Immunospheres (Dynabead® (a registered trademark owned by Dynal, Norway), 0.6 mg/ml) coated with monoclonal antibodies directed against a *Salmonella* common flagellar antigen.
- RV broth:: Rappaport-Vassiliadis broth (Merck 7700).
- RVS broth:: Rappaport-Vassiliadis broth (Oxoid CM 866).
- RM:: Raw medium: BPW supplemented with 17 g/l NaS₂O₃ (Merck 6516) of which a part is converted to tetrathionate by the addition of 19 ml/l Iodine solution.
- Iodine solution:: 6 g Iodine (Merck 4761) + 5 g KI (Merck 5043) + 20 ml H₂O.
- STOP:: Glycine buffer, 0.1 M, pH 10.5.
- SUB:: 4-methylumbelliferyl-β-galactosid, 100 µM, 1 mM MgCl₂ in 0.7% dimethylformamide.
- TSI:: Triple-Sugar-Iron (Difco 0265).
- TTB:: Tetrathionate broth (Difco 0104).
- Wash:: PBS (phosphate buffered saline) + 0.1% Triton-X100, pH 7.4.
- XLD:: Xylose Lysine desoxycholate agar (Gibco 152-05600/Oxoid CM 469).
- Lysine broth:: 2 g L-lysine (Merck 5700) dissolved in 10 ml H₂O and added to 200 ml of Decarboxylase Base Moeller (Difco 0890).
- Iron sulphite:: Agar medium prepared as described by Anonymous, 1980.
- MX4:: MB supplemented with 17 g/l Na-S₂O₃ (Merck 6516) of which a part is converted to tetrathionate by the addition of 20 µl/ml Iodine solution.

All enriched samples were determined by the use of an ELISA-technique (Enzyme Linked Immune Sorbent Assay) on an automated analyzer. The examples 3 - 8 were performed employing the EiaFoss™ Analyzer, an instrument designed by Foss Electric, Denmark, for automated ELISA analyses, whereas the experiments in example 1 and 2 were performed employing a development model of the same instrument.

### ELISA-1:

This ELISA analysis is based on the "sandwich-technique" using two different monoclonal antibodies directed against the *Salmonella* antigens. The antibodies react slightly with the native flagella, but strongly with a heat-treated extract of *Salmonella.* Therefore, the enriched samples are heat-treated at 100°C for 15 minutes as described in detail in example 1 before performing the ELISA. This heat-treatment kills/inactivates the *Salmonella* and other pathogenic bacteria. The ELISA is able to detect 10⁵ cells per ml. The enrichment is adapted to ensure that the number or *Salmonella* is at least 10⁵ per ml when enrichment is completed.

The automated ELISA analysis on the EiaFoss™ Analyzer takes approximately 75 minutes, but can be performed manually, and can be described as follows:
- One test tube with WASH (negative control) and one test tube with 500 µl calibrator (a heat-treated suspension of *Salmonella* typhimurium diluted to a concentration of approximately 10⁶ cells per ml) are placed in a sample disc together with test tubes containing 500 µl heat-treated samples.
- The samples are warmed up to 35°C and the analysis starts.
- 90 µl particle sol. 1 are added to each sample and incubated for 12½ minutes. *Salmonella* antigens present in the sample will react with antibody and form immune complexes on the surface of the immunospheres. The total surface area of the microspheres is very large, and this allows a fast and efficient binding between antibodies and antigens to take place.
- Unbound material is washed away with WASH while the immunospheres are retained in the side of the test tube by magnetic force.
- 130 µl conjugate 1 is then added and incubated 12½ minutes. The conjugated antibody binds to the immunosphere-antigen complex. This creates a "sandwich" with *Salmonella* antigens as filling.
- Unbound marked antibody is washed away with WASH.
- 200 µl SUB is added and incubated 12½ minutes. The enzyme bound to the particles will split the substrate to a.o. 4-methylumbelliferyl, a fluorescent compound which is measured by a fluorescence detector (excitation 365 nm, emission 450 nm).
- The enzyme reaction is stopped by adding 200 µl STOP. The concentration of 4-methylumbelliferyl is now measured by the detector and the concentration is proportional to the amount of *Salmonella* antigen present in the sample. The fluorescence signal for the individual sample is compared to a cut-off value, defined as 25% of the calibrator signal. Samples with a signal higher than or equal to the cut-off value are considered positive. Samples with a signal below the cut-off value are considered negative.

### ELISA-2:

This ELISA analysis is based on the "sandwich-technique", too, using two different antibodies (one monoclonal and one polyclonal) against the *Salmonella* antigens. The antibodies also react slightly with the native flagella, but strongly with a heat-treated extract of *Salmonella.* Therefore, the enriched samples are heat-treated at 100°C for 15 minutes as described in detail in example 1 before performing the ELISA. This heat-treatment kills/inactivates the *Salmonella* and other pathogenic bacteria. The ELISA is able to detect 10⁶ cells per ml.

The ELISA analysis can be described as follows:
- One test tube with WASH (negative control) and one test tube with 500 µl positive control (a purified solution of *Salmonella* typhimurium flagellin diluted to a concentration corresponding to a flagellin concentration in a cell suspension of approximately 10⁶ cells per ml) are placed in a sample disc together with test tubes containing 500 µl heat-treated samples.
- 60 µl particle sol. 2 which also contains 63 µg/ml conjugate 2 are added to each sample and incubated 25 minutes. *Salmonella* antigens present in the sample will react with antibody and form immune complexes on the surface of the immunospheres. The biotin-conjugated antibody simultaneously binds to the immunosphere-antigen complex. This creates a "sandwich" with *Salmonella* antigens as filling. The total area of the microspheres is very large, and this allows a fast and efficient binding between antibodies and antigens to take place.
- Unbound material is washed away with WASH while the immunospheres are retained in the side of the test tube by magnetic force.
- 100 µl AV-GAL is then added and incubated for 20 minutes. AV-GAL binds to the immunosphere-antigen-antibody complex.
- Unbound AV-GAL is washed away with WASH.
- 230 µl SUB is added and incubated for 8 minutes. The enzyme bound to the particles will split the substrate to a.o. 4-methylumbelliferyl, a fluorescent compound which is measured by a fluorescence detector (excitation 365 nm, emission 450 nm).
- The enzyme reaction is stopped by adding 230 µl STOP. The concentration of 4-methylumbelliferyl is now measured by the detector and the concentration is proportional to the amount of *Salmonella* antigen present in the sample. The fluorescence signal for the individual sample is compared to a cut-off value, defined as two times the signal of the negative control. Samples with a signal higher than or equal to the cut-off value are considered positive. Samples with a signal below the cut-off value are considered negative.

The antibodies employed in the above-described ELISAs may be provided by methods known to the person skilled in the art.

A polyclonal antibody against *Salmonella* can for instance be produced by immunising rabbits with purified flagella following a standardised immunisation procedure. The rabbits are bled and the serum is isolated and purified also using standard techniques. The antibodies are further purified by absorbtion of anti-serum comprising polyclonal antibodies using purified flagella from a cross-reacting *Citrobacter freundii.*

A monoclonal antibody may be produced according to standard monoclonal antibody techniques using *Salmonella* flagellin as immunogen (for information on monoclonal antibodies, cf. *i.a.* Köhler & Millstein, 1975).

### EXAMPLE 1

### Primary selective enrichment and determination of Salmonella in spiked samples of minced meat.

Two portions of 1 g of a Salmonella-free minced meat sample were transferred to two test tubes each containing 10 ml of RM. An overnight culture (37°C) of *Salmonella* was diluted 10⁵ times in a 0,85% NaCl solution, which resulted in a final concentration of *Salmonella* of about 3 X 10³ cells/ml. 50 µl of this cell suspension was added to one of the above-mentioned test tubes, giving rise to an average initial cell number of 15 cells per ml. Both test tubes were incubated for 24 hours at 41°C.

Following enrichment, the test tubes were heated in boiling water for 15 minutes. After cooling, the heat-treated suspensions were subjected to an ELISA-2 analysis.

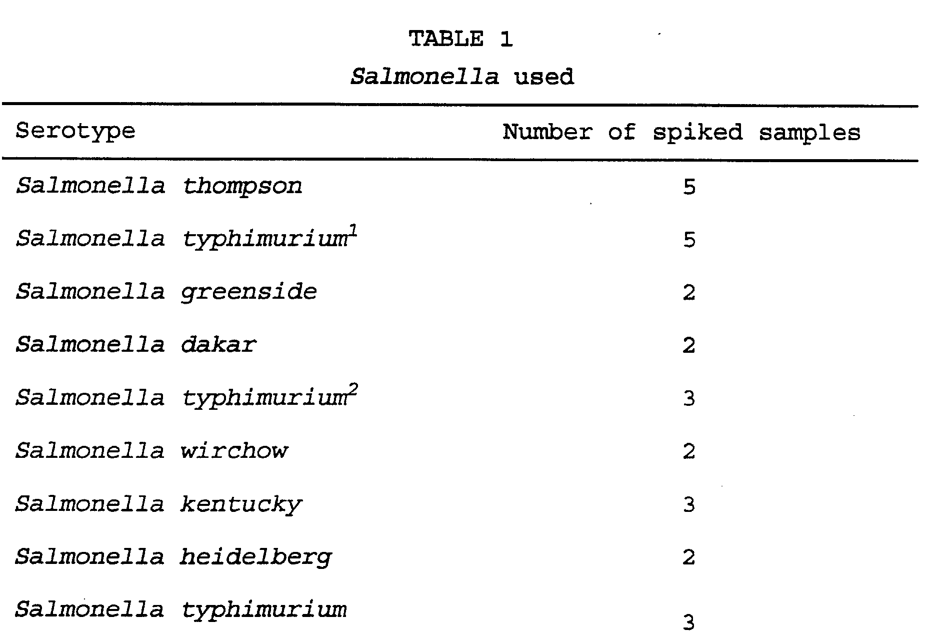

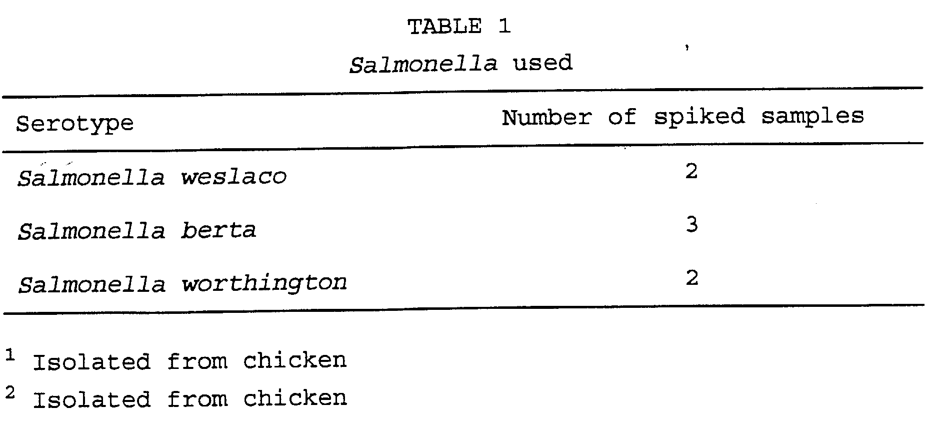

A total of 34 minced meat samples were tested as described above (the pattern of contamination is indicated in table 1). All 34 spiked samples showed positive reaction in the ELISA-2 analysis and none of the uncontaminated meat samples were found positive.

Thus, the method of the invention for enriching *Salmonella* from raw food samples exhibits the ability to specifically enrich *Salmonella* from a concentration of about 15 cells/ml to above the level corresponding to the cut-off value of the ELISA-2 which is about 10⁶ cells/ml.

### EXAMPLE 2

### Temperature dependency of the method of example 1

25 minced meat samples were tested in a similar manner to the test in example 1, with the exception that the primary selective enrichment was also performed at 37°C and 43°C. The scheme for the contamination of the meat samples was as listed in table 2.

**TABLE 2**

| *Salmonella* used | |
|---|---|
| Serotype | Number of spiked samples |
| *Salmonella thompson* | 2 |
| *Salmonella typhimurium*^{*1*} | 3 |
| *Salmonella typhimurium*^{*2*} | 3 |
| *Salmonella wirchow* | 2 |
| *Salmonella kentucky* | 3 |
| *Salmonella heidelberg* | 2 |
| *Salmonella typhimurium* | 3 |
| *Salmonella weslaco* | 2 |
| *Salmonella berta* | 3 |
| *Salmonella worthington* | 2 |

| | |
|---|---|
| ¹ Isolated from chicken | |
| ² Isolated from chicken | |

The results are listed in table 3:

**TABLE 3**

| Influence of temperature in primary selective enrichment | | | |
|---|---|---|---|
| | Temperature | | |
| | 37°C | 41°C | 43°C |
| Un-inoculated samples No. of positives | 1/25* | 0/25 | 0/25 |
| Inoculated samples No. of positives | 24/25 | 25/25 | 21/25 |

| | | | |
|---|---|---|---|
| * Considered as a false-positive ELISA-result. | | | |

Thus, as can be clearly seen from the results above, the temperature optimum seems to be about 41°C.

In order to further investigate the temperature dependency of the method, 9 samples of minced meat were tested as above at temperatures of 39°C, 40°C, 41°C and 42°C. The scheme of inoculation was as can be read from table 4:

**TABLE 4**

| *Salmonella* used | |
|---|---|
| Serotype | Number of spiked samples |
| *Salmonella thompson* | 3 |
| *Salmonella typhimurium*^{*1*} | 2 |
| *Salmonella greenside* | 2 |
| *Salmonella spp* | 2 |

| | |
|---|---|
| ¹ Isolated from chicken | |

The results of these tests are listed in table 5:

**TABLE 5**

| Influence of temperature in primary selective enrichment | | | | |
|---|---|---|---|---|
| | Temperature | | | |
| | 39°C | 40°C | 41°C | 42°C |
| Un-inoculated samples No. of positives | 0/9 | 0/9 | 0/9 | 0/9 |
| Inoculated samples No. of positives | 9/9 | 9/9 | 9/9 | 9/9 |

As can be clearly seen, the method of example 1 is highly sensitive and highly specific in the total range of 39°C-42°C.

### EXAMPLE 3

### Selectivity of the primary selective enrichment for Salmonella from raw and processed products.

25 different *Salmonella* strains and 11 different non-*Salmonella* strains were enriched under non-selective conditions in MB (37°C, 18 hours), heat-treated for 15 minutes at 100°C and subsequently serially diluted 10-fold to 10.000 times dilution. The 11 non-*Salmonella* strains were selected from 100 strains closely related to *Salmonella* and all showed (in an earlier experiment) weak to strong cross-reactivity in ELISA-1 whereas the remaining 89 strains did not. The diluted suspensions were subjected to ELISA-1. The results are shown in table 6:

**TABLE 6**

| Percentage ELISA-1-positives | | | | | |
|---|---|---|---|---|---|
| | Dilutions | | | | |
| Bacteria | 10⁻⁴ | 10⁻³ | 10⁻² | 10⁻¹ | 10⁰* |
| Salmonella | 64% | 84% | 96% | 100% | 100% |
| non-Salmonella | 0% | 37% | 45% | 82% | 100% |

| | | | | | |
|---|---|---|---|---|---|
| * 10⁰ dilution corresponds to a cell count of approximately 2 x 10⁹ cells/ml. | | | | | |

The same strains of *Salmonella* and non-*Salmonella* were also subjected to ELISA-1 subsequent to a primary selective enrichment particularly suitable for the enrichment of raw products:

To a test tube containing 10 ml RM supplemented with 1.5 µg/ml novobiocin and 1 g of heat-treated (100°C for 15 minutes) minced meat approximately 10³ cells per ml were added. The test tube was then incubated at 41°C for 19 hours. Then 0.5 ml enriched suspension was transferred to 10 ml MBN which was then incubated for 3 hours at 42°C. The MBN was heat-treated, diluted and ELISA-1-tested as described above in this example.

**TABLE 7**

| Percentage ELISA-1-positives | | | | | |
|---|---|---|---|---|---|
| | Dilutions | | | | |
| Bacteria | 10⁻⁴ | 10⁻³ | 10⁻² | 10⁻¹ | 10⁰* |
| Salmonella | 0% | 44% | 96% | 96% | 96% |
| non-Salmonella | 0% | 0% | 9% | 9% | 9% |

| | | | | | |
|---|---|---|---|---|---|
| * 10⁰ dilution corresponds to a cell count of approximately 5 x 10⁸ cells/ml. | | | | | |

Thus, as is evident from tables 6 and 7, the enrichment according to the invention is capable of removing any cross-reactivity of 10 of the 11 non-*Salmonella* strains which cross-reacted when enriched non-selectively. And, as can be seen, the *Salmonella* were to a much lesser degree affected by the selective primary selective enrichment.

The same strains of *Salmonella* and non-*Salmonella* were also subjected to ELISA-1 after a primary selective enrichment which in particular is suitable for the enrichment of processed products:

Approximately 30 cells per ml were added to a test tube containing 10 ml of BPW supplemented with 2.5 µg/ml novobiocin. The test tube was then incubated at 37°C for 4 hours. Immediately thereafter, the test tube was further incubated at 41°C for 15 hours. Then, 0.5 ml enriched suspension was transferred to 10 ml MBN which was then incubated for 3 hours at 42°C. The MBN was heat-treated, diluted and ELISA-1 tested as described above in this example.

**TABLE 8**

| Percentage ELISA-1-positives | | | | | |
|---|---|---|---|---|---|
| | Dilutions | | | | |
| Bacteria | 10⁻⁴ | 10⁻³ | 10⁻² | 10⁻¹ | 10⁰* |
| Salmonella | 0% | 80% | 88% | 96% | 96% |
| non-Salmonella | 0% | 0% | 9% | 9% | 18% |

| | | | | | |
|---|---|---|---|---|---|
| * 10⁰ dilution corresponds to a cell count of approximately 5 x 10⁸ cells/ml. | | | | | |

Thus, as is evident from comparing tables 6 and 8, the enrichment according to the invention is capable of removing any cross-reactivity of 9 of the 11 non-*Salmonella* strains which cross-reacted when enriched non-selectively. However, the *Salmonella* were to a much lesser degree affected by the selective primary selective enrichment.

### EXAMPLE 4

### Comparison of a standard cultural procedure and the primary selective enrichment and determination of Salmonella in raw products.

A total of 54 raw samples (e.g. broilers, minced meat, egg white and egg yolk) were investigated for the presence of *Salmonella* using a standard cultural protocol utilising the enrichment procedures proposed by Nordic Committee on Food Analysis (NMKL no. 71) with the main exception that the RV was incubated at 42.5°C instead of 41.5°C:

A sample of 25 g was mixed with 225 ml BPW in sterile stomacher bags and homogenized by hand for 15-20 seconds. The bags were incubated at 37°C ± 1°C for 18-24 hours. A portion of 0.1 ml was transferred to 10 ml of RV broth and incubated in a water bath at 42.5 ± 0.2°C for 20-24 hours. From the enriched RV broth a loopful was streaked onto BGA. Agar plates were incubated at 37 ± 1°C and inspected after 18-24 hours and rechecked after 48 hours. Four to six suspect colonies from each plate, if available, were inoculated into TSI, iron sulphite agar, lysine broth, ONPG broth and finally confirmed serologically by agglutination with polyvalent O- and H-serum. Still dubious isolates were further identified using the API-20E system.

The 54 samples were tested in parallel using a procedure according to the invention:

A sample of 25 g was mixed and homogenized as described for the standard cultural method described above. The raw products were mixed with 225 ml of RM supplemented with 1.5 µg/ml novobiocin. The RM with raw samples was incubated in a water bath at 41.5 ± 0.2°C for 19-24 hours. The samples were post-enriched for 3 hours in MBN and tested employing the ELISA-1-technique described in Example 3 above. Positive samples were verified by transferring 0.1 ml from RM to 10 ml RV broth which were incubated in a water bath at 42.5 ± 0.2°C for 20-24 hours. From the RV broth a loopful was streaked onto BGA, which was incubated at 37 ± 1°C for 18-24 hours. suspect colonies (one to three from each dish) were inoculated in TSI and LIA and confirmed serologically with polyvalent O- and H-serum. Still dubious isolates were further identified using the API-20E system. When an ELISA-1-positive sample could not be verified by the above procedure, isolation of *Salmonella* was also attempted using TTB as selective enrichment broth. From RM, 1 ml was transferred to 10 ml TTB and incubated at 42.5°C for 18-24 hours, followed by subcultivation on BGA and XLD. Suspect colonies were examined as described above.

The results are presented in table 9:

**TABLE 9**

| Number of *Salmonella* positives | | |
|---|---|---|
| Total number of samples | Positive by the standard cultural method | Confirmed positive by the method according to the invention |
| 54 | 18 | 21 |

Hence, the combined method according to the invention is as least as sensitive in detecting *Salmonella* as the standard cultural method. No false positives were observed utilising the method according to the invention which indicates that the primary selective enrichment method is highly specific.

### EXAMPLE 5

### Primary selective enrichment and determination of Salmonella in naturally contaminated samples of meat and bone meal and dried blood albumin using the method M1.

A total of 60 samples comprising 45 samples of meat and bone meal and 15 samples of dried blood albumin were investigated for the presence of *Salmonella* as follows:

### M1:

From each sample a 10 g portion was transferred to 160 ml BPW. The BPW with the sample was incubated at 37°C for 4 hours. Then the temperature was adjusted to 41°C and the incubation was continued for 15 hours. From each enrichment broth a portion of 0.5 ml was transferred to 10 ml of MBN and incubated at 42°C for 3 hours. The MBN was then heated in boiling water for 15 minutes and cooled under tap water prior to conducting an ELISA-1 assay. ELISA positive samples were verified by transferring 0.1 ml from the primary enrichment broth (BPW) to 10 ml RV broth and 1.0 ml to 10 ml TTB. The tubes containing RV and TTB were incubated at 43°C for 20-24 hours. From RV and TTB a loopful was streaked onto BGA and XLD which were incubated at 37°C for 18-24 hours. Suspect colonies were inoculated in TSI and LIA and confirmed serologically with polyvalent O-serum and H-serum. Still dubious isolates were further identified using the API-20E system.

Further, the samples were investigated using a standard cultural protocol:

### Standard protocol:

10 g from each sample was transferred to 160 ml BPW and incubated at 37°C for 18-24 hours. Then portions of 0.1 ml and 1.0 ml of the enrichment broth were transferred to 10 ml RV broth and 1.0 ml to 10 ml TTB, respectively. The tubes containing RV and TTB were incubated at 43°C for 20-24 hours. From RV and TTB a loopful was streaked onto BGA and XLD which were incubated at 37°C for 18-24 hours. Suspicious colonies were inoculated in TSI and LIA and confirmed serologically with polyvalent O-serum and H-serum. Still dubious isolates were further identified using the API-20E system.

The results are presented in table 10

**TABLE 10**

| Number of *Salmonella* positives | | |
|---|---|---|
| Total number of samples | Confirmed ELISA-1 positives using M1 | Positives using the standard cultural protocol |
| 60 | 31 | 26 |

As is apparent from the above table, the method M1 is more efficient in detecting *Salmonella* in the investigated samples of processed products than the standard cultural protocol. It is remarkable that no false positives have been observed using M1.

Thus, the selectivity which is introduced by elevating the temperature improves the *Salmonella* detection when employing the ELISA technique as well as when employing a traditional plating technique (*i.e.* the confirmation cultural protocol).

### EXAMPLE 6

### Temperature dependency of the M1-method of example 5.

63 processed food and feed samples were tested in a manner similar to M1 in example 5, with the exceptions that the primary selective enrichment was also performed at 40°C and 42°C, RVS was used instead of RV and incubated at 42°C and TTB was also incubated at 42°C.

18 of the samples tested were feeds in which *Salmonella* had been isolated previously. The remaining 45 samples, comprising 9 samples of blanched vegetables, 7 samples of cocoa, 5 samples of pet food, 4 samples of crustaceans, 9 samples of prepared meals, 9 samples of soups and 2 samples of desiccated coconut, had not been investigated previously. The results are reported in table 11:

**TABLE 11**

| Confirmed ELISA-1 positives using M1 | | | |
|---|---|---|---|
| | Temperature | | |
| Total number of samples | 40 °C | 41°C | 42°C |
| 63 | 10 | 12 | 11 |

Hence, the M1 enrichment method is uniformly efficient with respect to detect *Salmonella* in processed products in the temperature range from 40°C to 42°C. As in example 5, no false positives have been observed.

### EXAMPLE 7

### Primary selective enrichment and determination of Salmonella in naturally contaminated samples of meat and bone meal and dried blood albumin using the methods M2 and M3.

A total of 89 feed samples of which a few by the supplier were reported to be contaminated with *Salmonella* were investigated for the presence of *Salmonella* as follows:

### M2:

From each sample a 10 g portion was transferred to 160 ml BPW supplemented with 2.5 µg/ml µg novobiocin and was incubated at 37°C for 4 hours. Then the temperature was adjusted to 41°C and the incubation was continued for 15 hours. From each enrichment broth a portion of 0.5 ml was transferred to 10 ml of MX4 and post-enriched at 41°C for 4 hours. The MX4 was then heated in boiling water for 15 minutes and cooled under tap water prior to conducting an ELISA-1 assay. ELISA positive samples were verified by transferring 0.1 ml from the primary enrichment broth (BPW) to 10 ml RVS broth and 1.0 ml to 10 ml TTB. The tubes containing RVS and TTB were incubated at 42°C and 43°C, respectively, for 20-24 hours. From RVS and TTB a loopful was streaked onto BGA and XLD which were incubated at 37°C for 18-24 hours. Suspect colonies were inoculated in TSI and LIA and confirmed serologically with polyvalent O-serum and H-serum. Still dubious isolates were further identified using the API-20E system.

### M3:

A similar *modus operandi* as in M2 was employed for another batch of the same samples with the exception that the incubation temperature was elevated to 43°C instead of 41°C. Finally, the samples were investigated using a standard cultural protocol:

10 g from each sample was transferred to 160 ml BPW and incubated at 37°C for 18-24 hours. Then portions of 0.1 ml and 1.0 ml of the enrichment broth were transferred to 10 ml RVS broth and 1.0 ml to 10 ml TTB, respectively. The tubes containing RVS and TTB were incubated at 42°C and 43°C, respectively, for 20-24 hours. From RVS and TTB a loopful was streaked onto BGA and XLD which were incubated at 37°C for 18-24 hours. Suspect colonies were inoculated in TSI and LIA and confirmed serologically with polyvalent O-serum and H-serum. Still dubious isolates were further identified using the API-20E system.

The results are presented in table 12:

**TABLE 12**

| Number of *Salmonella* positives | | | |
|---|---|---|---|
| Total number of samples | Confirmed ELISA-1 positives using M2 | Confirmed ELISA-1 positives using M3 | Positives using the standard cultural protocol |
| 89 | 6 | 4 | 4 |

The method M2 detects *Salmonella* in 50% more samples than the standard protocol. By increasing the temperature-level to 43°C (M3) instead of 41°C (M2) the number of positive samples is reduced to a level comparable to that of the standard protocol. However, 2 false-positive samples are detected when employing M2 while only one false-positive is detected by M3. Hence, the increased incubation temperature results in a increased selectivity, but a decreased sensitivity.

### EXAMPLE 8

### Primary selective enrichment and determination of Salmonella in spiked samples of spices.

A total of 8 samples of spices (pepper, curry, paprika, powdered mustard, tandoori) was investigated as follows: Two portions of 6.25 g of each spice-sample were transferred to 2 flasks each containing 200 ml of BPW supplemented with 2.5 µg/ml novobiocin. To one of the portions a capsule containing milk powder contaminated with about 5 CFU (colony forming units) *Salmonella panama* (the capsules were obtained from the National Institute of Public Health and Environmental Protection, Bildhoven, The Netherlands). All flasks were incubated at 37°C for 4 hours whereafter the incubation was continued for 15 hours at 41°C. Then 1 ml was transferred to 10 ml MBN and incubated 3 hours at 42°C and subsequently heat-treated in boiling water for 50 minutes. After cooling the samples were subjected ELISA-1. All samples (both ELISA positives and negatives) were verified by transferring 0.1 ml from the primary enrichment broth (BPW) to 10 ml RVS broth. The tubes were incubated at 42°C for 20-24 hours. A loopful was streaked onto BGA and XLD which were incubated at 37°C for 18-24 hours. Suspect colonies were inoculated in TSI and LIA and confirmed serologically with polyvalent O-serum and H-serum. Still dubious isolates were further identified using the API-20E system.

Results are given in table 13:

**TABLE 13**

| Number of *Salmonella* positives | | |
|---|---|---|
| Total number of samples | Inoculated samples Confirmed ELISA-positives | Un-inoculated samples Confirmed ELISA-positives |
| 8 | 7 | 0 |

As a conclusion, the method detects *Salmonella panama* in 7 of 8 spiked samples of spices. In the remaining spiked sample *Salmonella* could not be detected by neither ELISA nor by the confirmation procedure. However, it is not inconceivable that this indicates that the capsule was deprived of Salmonella. One of the un-inoculated samples (12.5%) gave a false-positive result by the ELISA.

### REFERENCES

Anonymous, 1980, Determination of the number of sulphite-reducing clostridia in foods. Nordic committee on food analysis, Helsinki. Method no. 56.

Beumer R R, Brinkman E, Rombouts F M, 1991, Int. J. Food Microbiol. **12**, pp. 363-374.

Cowan S T, 1974, Cowan and Steel's Manual for the identification of medical bacteria. Cambridge University Press, Cambridge, Second ed. p. 153.

D'Aoust J-Y, Sewell A M, 1988a, J. Food Protect. **51**, pp. 853-856.

D'Aoust J-Y, Sewell A M, 1988b, J. Food Protect. **51**, pp. 538-541.

D'Aoust J-Y, Sewell A M, Greco P, 1991, J. Food Protect. **54**, pp. 725-730.

Emswiler-Rose B, Gehle W D, Johnston R W, Okrend A, Moran A, Bennett B., 1991, J. Food Sci. **49**, pp. 1018-1020.

Entis P, 1986, Membrane filtration systems. In: Pierson M D and Stern N J (Eds.), Foodborne Microorganisms and their toxins: Developing methodology. Marcel Dekker, New York, NY, and Basel, pp. 91-106.

Hadfield S Q, Lane A, Mcillmurray M B, 1987, J. Immun. Met. **97**, pp. 153-158.

Holbrook R, Anderson J M, Baird-Parker A C, Dodds L M, Sawhney D, Stuchbury S H, Swaine D, 1989, Lett. Appl. Microbiol. **8**, pp. 139-142.

Köhler & Millstein, Nature **256**, 1975, p. 495.

Nielsen P E *et al.,* 1991, Science **254**, pp. 1497-1500.

Notermans S, Werrmars K, 1991, In. J. Food Microbiol. **12**, pp. 91-102.

Smith P J, Boardman A, Shutt P C, 1989, J. Appl. Bacteriol. **67,** pp. 575-588.

Vassiliadis P, 1983, J. Appl. Bacteriol. **54,** pp. 69-76.

Wolcott M J, 1991, J. Food Protect. **54**, pp. 387-401.

## Claims

1. A method for the determination of bacteria belonging to the genus *Salmonella* in a sample,
the method comprising an enrichment procedure which comprises either
1) transferring the sample to an aqueous growth medium for *Salmonella,* the growth medium being buffered to a pH between 5.6 and 9.3, tetrathionate and/or another selective substance different from selenite being added to or generated in the medium before, simultaneously with or after the transfer, and keeping the resulting mixture at a temperature of at the most 38°C for a period of time of between 10 minutes and 8 hours after the transfer of the sample (pre-enrichment period) and then increasing the temperature of the mixture to 39-43°C and keeping the mixture at the increased temperature of 39-43°C for a period of time after the increase of the temperature (enrichment period), the sum of the periods of time of the pre-enrichment period and the enrichment period being between 10-48 hours, or
2) transferring the sample to an aqueous growth medium for *Salmonella,* the growth medium being buffered to a pH between 5.6 and 9.3, the growth medium with the sample being kept at a temperature of 39-42.5°C, tetrathionate and/or another selective substance different from selenite being added to or generated in the medium before, simultaneously with or at the most 8 hours after the transfer, and keeping the resulting mixture at a temperature of 39-42.5°C for a period of time of between 10-48 hours from the transfer (enrichment period),
and then performing a determination to identify and/or quantify *Salmonella* present in the enrichment medium after the proliferation, the determination comprising an assay step which is based on the identification and/or quantification of a target molecule or a molecular interaction involving a target molecule and which is different from traditional agar plating techniques,
the conditions of the enrichment procedure being so adapted to the determination that a test (test 1)
comprising the enrichment procedure and the determination wherein the selective enrichment medium has been inoculated with a strain of *Salmonella* to a starting concentration of about 15 cells/ml and 10% randomly selected minced raw meat has been added to the enrichment medium at the beginning of the enrichment period and the resulting mixture has been maintained at the temperature or the temperatures for the enrichment period
will produce, in the determination, a positive result in at least 90% of all cases when testing a panel of randomly chosen *Salmonella* serotypes, and a test (test 2)
comprising the enrichment procedure and the determination wherein the selective enrichment medium has been inoculated at the beginning of the enrichment period with a strain of one of the non-*Salmonella* species
*Citrobacter freundii,*
*Escherichia coli,* and
*Enterobacter cloacae,*
to a starting concentration thereof of about 100 cells/ml, the selective enrichment medium being substantially free from microorganisms other than the non-*Salmonella* strain inoculated, and the resulting mixture has been maintained at the temperature or the temperatures for the enrichment period
will give rise to a percentage of false positives in the assay of at the most 15% within each species when testing a panel of 20 strains randomly selected from the non-*Salmonella* species.

2. A method according to claim 1, wherein the temperature is in the range between 40 and 42°C, preferably between 40.5 and 41.5°C, during at least a substantial part of the enrichment period.

3. A method according to claim 1 or 2, wherein the tetrathionate and/or another selective substance different from selenite is added to or generated in the medium at the most 4 hours, such as at the most 2 hours, at the most 1 hour, and at the most 10 minutes after the transfer.

4. A method according to any of the preceding claims, variant 2), wherein the only selective substance which is being added to or generated in the medium before, simultaneously with or after the transfer is novobiocin.

5. A method according to claim 4, wherein the concentration of novobiocin in the resulting mixture is in the range of about 1 to 100 µg per ml, such as in the range of about 1-20 and 2-13 µg per ml.

6. A method according to any of claims 1-3, variant 1), wherein the duration of the pre-enrichment period is in the range of from ½-7 hours, such as in the range of 1-6½ hours, 2-6 hours, and 3-5 hours, and preferably the duration is about 4 hours.

7. A method according to any of the preceding claims, wherein the sum of the time of the enrichment procedure and the determination step has a duration of at most 56 hours, such as a duration of at most 48 hours, 40 hours, 34 hours, 32 hours, 28 hours, 24 hours, 22 hours, and 20 hours.

8. A method according to any of the preceding claims, wherein the duration of the enrichment period or, with respect to variant 1), the sum of the pre-enrichment period and the enrichment period, is 15-30 hours, such as 17-24 hours.

9. A method for the determination of bacteria belonging to the genus *Salmonella* in a sample, the method comprising
an enrichment procedure which comprises transferring the sample to an aqueous growth medium for *Salmonella,* the growth medium being buffered to a pH between 5.6 and 9.3, tetrathionate being added to or generated in the medium before, simultaneously with or at the most 3 hours after the transfer, and keeping the resulting mixture at a temperature of about 36-39°C for a period of time of between 15-30 hours after the transfer of the sample (enrichment period),
and then performing a determination to identify and/or quantify *Salmonella* present in the enrichment medium after the proliferation, the determination comprising an assay step which has a duration of at most 7 hours and which is based on the identification and/or quantification of a target molecule or a molecular interaction involving a target molecule and which is different from traditional agar plating techniques,
the sum of the time of the enrichment procedure and the determination having a duration of at most 40 hours and the conditions of the enrichment procedure being so adapted to the determination that test 1 as defined in claim 1 will produce, in the determination, a positive result in at least 90% of all cases when testing a panel of randomly chosen *Salmonella* serotypes, and that test 2 as defined in claim 1 will give rise to a percentage of false positives in the assay of at the most 15% within each species when testing a panel of 20 strains randomly selected from the non-*Salmonella* species.

10. A method according to claim 9, wherein the sum of the time of the enrichment procedure and the determination step has a duration of at most 34 hours, such as a duration of at the most 32 hours, 28 hours, 24 hours, 22 hours, and 20 hours.

11. A method according to claim 9 or 10, wherein the duration of the enrichment period is 17-24 hours.

12. A method according to any of claims 9-11, wherein the temperature is in the range between 36°C and 39°C during at least a substantial part of the enrichment period.

13. A method according to any of claims 9-12, wherein the tetrathionate is added to or generated in the medium at the most 1 hour after the transfer, such as after at the most 5 minutes.

14. A method according to any of the preceding claims, which is calibrated against the standard protocol NMKL no. 71 (Nordic Committee on Food Analysis method no. 71, 1991, fourth edition, seventeenth printing), with the exceptions that Oxoid CM 866 Rappaport-Vassiliadis broth (RVS) is used instead of Merck 7700 Rappaport-Vassiliadis broth (RV) and RVS is incubated at 42°C and that Gibco 152-05600/Oxoid CM 469 Xylose Lysine desoxycholate agar (XLD) is also used as a plating medium, in such a manner that the determination results in at least 80% true positive results compared to the standard protocol, and gives rise to at the most 10% of false positive results when testing 100 samples of minced meat (test 3), and the weight of the portion of sample to be tested by each of the two methods being 25 g, the 100 samples being provided from 10 different retails, each of which delivers 10 different samples, 20 of the samples provided having been inoculated with about 15 *Salmonella typhimurium* cells per sample and 20 of the samples provided having been inoculated with about 15 *Salmonella enterititis* cells per sample.

15. A method according to any of the preceding claims, wherein the sample is different from a processed product.

16. A method according to claim 14 or 15, wherein the determination gives rise to at least 85% true positive results compared to the standard protocol in test 3, such as at the least 90%, 92%, 95%, 97%, 98%, 99%, and 100% true positive results.

17. A method according to claim 16, wherein the determination gives rise to at least 101% true positive results compared to the standard protocol in test 3.

18. A method according to any of claims 14-17, wherein the determination gives rise to at most 8% false positive results in test 3, such as at most 5%, 3%, 2%, and 1% false positive results.

19. A method according to claim 18, wherein the determination gives rise to 0 false positive results in test 3.

20. A method according to any of the preceding claims, wherein the determination gives rise to at least 92% true positive results in all cases of test 1, such as at least 95%, 97%, 98%, 99%, 99.5%, and 99.9% true positive results.

21. A method according to any of the preceding claims, wherein the determination gives rise to at most 12% false positive results in test 2, such as at most 10%, 7%, 5%, 3%, 2%, 1%, and ½% false positive results.

22. A method according to any of the preceding claims, wherein the concentration of tetrathionate in the resulting mixture is in the range of about 5-40 mM, such as in the range of about 7-35 mM, 9-30 mM, 11-28 mM, 13-26 mM, 14-25 mM, 15-23 mM, 16-22 mM, 17-21 mM, and 18-20 mM.

23. A method for the determination of bacteria belonging to the genus Salmonella in a sample,
comprising performing an enrichment procedure which comprises either
a) transferring the sample to an aqueous growth medium for *Salmonella,* the growth medium being buffered to a pH between 5.6 and 9.3 and containing substantially no bacterial growth-inhibiting substance, and keeping the resulting mixture at a temperature of at most 39.0°C for a period of time of at least 1 minute (pre-enrichment period), and then
increasing the temperature of the mixture to 39.5-43°C and optionally adding novobiocin and/or another selective substance different from selenite
and keeping the mixture at the increased temperature of 39.5-43°C for a period of time from the temperature increase (selective enrichment period), or
b) transferring the sample to an aqueous growth medium for *Salmonella,* the growth medium being buffered to a pH between 5.6 and 9.3, novobiocin and/or another selective substance different from selenite being added to the medium before, simultaneously with or after the transfer, and keeping the resulting mixture at a temperature of at most 39.0°C for a period of time of at least ½ hour from the transfer or the addition of novobiocin or another selective substance different from selenite, whichever the later (pre-enrichment period), and then
increasing the temperature of the mixture to 39.5-43°C and optionally adding novobiocin or another selective substance different from selenite
and keeping the mixture at the increased temperature of 39.5-43°C for a period of time from the temperature increase (selective enrichment period),
and then performing a determination to identify and/or quantify Salmonella present in the enrichment medium after the proliferation, the sum of the periods of time of the pre-enrichment period and the selective enrichment period being between 10-48 hours.

24. A method according to claim 23, wherein the duration of the pre-enrichment period is at least ½ hour, such as 1-10 hours, 1-8 hours, 2-6 hours, 3-5 hours, and 3½-4½ hours, and preferably the duration is about 4 hours.

25. A method according to claim 23 or 24, wherein the sum of the time of the enrichment procedure and the determination step has a duration of at most 56 hours, such as a duration of at most 48 hours, 40 hours, 32 hours, 28 hours, 24 hours, 22 hours, and 20 hours.

26. A method according to any of claims 23-25, wherein the sum of the time of the pre-enrichment period and the time of the selective enrichment period is 15-30 hours, such as 17-24 hours.

27. A method for the determination of bacteria belonging to the genus Salmonella in a sample, comprising performing an enrichment procedure which comprises
transferring the sample to an aqueous growth medium for *Salmonella,* the growth medium being buffered to a pH between 5.6 and 9.3, novobiocin being added to the medium before, simultaneously with or after the transfer, and keeping the resulting mixture at a temperature of at most 39.0°C for a period of time of between 15-30 hours from the transfer, the addition of novobiocin or another optional selective substance different from selenite, whichever the later (enrichment period),
and then performing a determination which has a duration of at the most 7 hours to identify and/or quantify Salmonella present in the enrichment medium after the proliferation,
the sum of the time of the enrichment procedure and the determination having a duration of at most 40 hours.

28. A method according to claim 27, wherein the sum of the time of the enrichment procedure and the determination step has a duration of at most 32 hours, such as a duration of at most 28 hours, 24 hours, 22 hours, and 20 hours.

29. A method according to claim 27 or 28, wherein the duration of the enrichment period is 17-24 hours.

30. A method according to any of claims 23-29, wherein the determination comprises an assay step which is based on the identification and/or quantification of a target molecule or a molecular interaction involving a target molecule and which assay step is different from agar plating.

31. A method according to any of claims 23-30, wherein the conditions of the enrichment procedure are so adapted to the determination that
- a test (test 4) comprising the enrichment procedure and the determination wherein the aqueous growth medium has been inoculated with heat-treated cells of a strain of *Salmonella* to a starting concentration of about 1000 heat-treated cells/ml and 5% non-fat dry milk has been added to the medium at the beginning of the enrichment procedure will produce, in the determination, a positive result in 90% of all cases when testing a panel of randomly chosen *Salmonella* serotypes, and
- a test (test 5) comprising the enrichment procedure and the determination, wherein the aqueous growth medium has been inoculated with a strain of one of the non-Salmonella species
*Citrobacter freundii,*
*Escherichia coli*, and
*Enterobacter cloacae,*
to a starting concentration thereof of about 100 cells/ml at the beginning of the enrichment procedure will give rise to a percentage of false positives in the determination of at the most 25% within each species of a panel of 20 randomly selected species of the non-Salmonella strains.

32. A method according to any of claims 23-31, which is calibrated against the standard cultural protocol NMKL no. 71 (Nordic Committee on Food Analysis method no. 71, 1991, fourth edition, seventeenth printing) in such a manner that the determination results in at least 80% true positive results compared to the standard protocol, and gives rise to at the most 10% of false-positive results when testing (test 6) a total of 50 samples of 50 g of milk powder randomly chosen with respect to batch and type, each sample being divided in a set of two parts of 25 g, one part of each set being subjected as sample to the method according to any of claims 23-31 and the other part of each set being subjected as sample to the standard cultural protocol, 25 of the 50 sets of 25 g parts being spiked in the following manner:
- one 25 g part of the set, subjected to the standard cultural protocol, is supplemented by adding, at the beginning of the enrichment procedure of the protocol, 0.1-1 g non-fat dry milk containing about 5 CFU *Salmonella panama* or *Salmonella typhimurium* to the aqueous growth medium used in the protocol,
- the other 25 g part of the set, subjected to the method according to any of claims 23-31, is supplemented by adding, at the beginning of the enrichment procedure, 0.1-1 g non-fat dry milk containing about 5 CFU *Salmonella panama* or *Salmonella typhimurium* to the aqueous growth medium.

33. A method according to claim 32, wherein the determination gives rise to at least 85% true positive results compared to the standard protocol in test 6, such as at least 90%, 92%, 95%, 97%, 98%, 99%, and 100% true positive results.

34. A method according to claim 33, wherein the determination gives rise to at least 101% true positive results compared to the standard protocol in test 6.

35. A method according to any of claims 32-34, wherein the determination gives rise to at most 8% false positive results in test 6, such as at most 5%, 3%, 2%, and 1% false positive results.

36. A method according to claim 35, wherein the determination gives rise to 0 false positive results in test 6.

37. A method according to any of claims 23-36, wherein the determination gives rise to at least 92% true positive results in all cases of test 4, such as at least, 95%, 97%, 98%, 99%, 99.5%, and 99.9% true positive results.

38. A method according to any of claims 23-37, wherein the determination gives rise to at most 20% false positive results in test 5, such as at most 15%, 10%, 6%, 3%, 2%, 1%, ½%.

39. A method according to any of the preceding claims, wherein the sample is taken from an animal, including a human being.

40. A method according to claim 39, wherein the origin of the sample is selected from the group consisting of faeces, gastric juice, urine, blood, lymph, cerebrospinal fluid, and a biopsy.

41. A method according to any of claims 1-38, wherein the sample is of environmental origin.

42. A method according to claim 41, wherein the origin of the sample is selected from the group consisting of dust, swaps, and fluff.

43. A method according to any of claims 23-38, wherein the sample is a processed product.

44. A method according to any of claims 1-38 and 43, wherein the sample is taken from a food or a feed.

45. A method according to any of the preceding claims, wherein the determination step comprises an immunological reaction.

46. A method according to claim 45, wherein the determination step comprises an assay selected from the group consisting of
an immune assay, such as a Radio Immune Assay (RIA), an Enzyme Immune Assay (EIA), an Enzyme Linked Immune Sorbent Assay (ELISA), a Fluorescence Antibody technique (FA), and an immune assay comprising the use of liposomes, micelles or liposome-like particles in the detection phase;
an assay involving immune immobilisation;
an assay involving immune agglutination such as latex agglutination;
a DNA/DNA-hybridisation assay;
a RNA/RNA-hybridisation assay; and
a DNA/RNA-hybridisation assay.

47. A method according to any of claims 1-44, wherein the determination step comprises a polymerase chain reaction (PCR).

48. A method according to any of claims 1-44, wherein the determination step comprises the use of peptide nucleic acids (PNA).

49. A method according to any of claims 1-44, wherein the determination step comprises the use of lectines.

50. A method according to any of claims 1-44, wherein the determination step comprises the use of bacteriophages, such as the bacteriophage Felix 01.

51. A method according to any of claims 1-44, wherein the determination step comprises a metabolic conversion by the *Salmonella* of a substance which results in a change in the electric resistance or impedance of the medium containing the *Salmonella* and wherein the detection of the *Salmonella* is accomplished by measuring this change.

52. A method according to any of claims 1-44, wherein the determination step comprises an assay for the ability of *Salmonella* to migrate on or through an agar matrix.

53. A method according to any of claims 1-44, wherein the determination step comprises an assay involving a filtration step, wherein the bacteria are separated from the medium and are retained on a filter, such as an assay involving a hydrophobic grid membrane filter method (HGMF).

54. A method according to any of claims 1-44, wherein the determination step comprises an assay involving a radiometric method.

55. A method according to any of claims 1-44, wherein the determination step comprises an assay involving a chromatographic separation.

56. A method according to any of the preceding claims, wherein the determination step involves a selective post-enrichment.

57. A method according to claim 56, wherein the selective post-enrichment has a duration of 2-6 hours, such as a duration of 2.0-4.5 hours, 2.5-3.5 hours, and preferably about 3 hours.

58. A method according to any of the preceding claims, wherein the aqueous growth medium for *Salmonella* have a concentration of selenite corresponding to at the most the selenite concentration in a 0.5% w/v sodium hydrogen selenite solution, preferably a selenite concentration of 0% w/v.

## Patentansprüche

1. Verfahren für die Bestimmung von Bakterien, die der Gattung Salmonella angehören, in einer Probe,
wobei das Verfahren ein Anreicherungsverfahren enthält, das entweder folgenden Schritt enthält
1) Übertragung der Probe auf ein wäßriges Wachstumsmedium für Salmonella, wobei das Wachstumsmedium auf einen pH zwischen 5,6 und 9,3 gepuffert ist, wobei Tetrathionat und/oder eine andere selektive Substanz, die nicht Selenit ist, dem Medium vor, gleichzeitig mit oder nach der Übertragung hinzugefügt wird oder in diesem erzeugt wird, und wobei die so erhaltene Mischung bei einer Temperatur von höchstens 38°C für eine Zeitspanne von 10 Minuten bis 8 Stunden nach der Übertragung der Probe (Voranreicherungsperiode) gehalten wird und die Temperatur der Mischung dann auf 39-43°C erhöht wird und die Mischung bei der erhöhten Temperatur von 39-43°C für eine Zeitspanne nach dem Anstieg der Temperatur (Anreicherungsperiode) gehalten wird, wobei die Summe der Zeitspannen der Voranreicherungsperiode und der Anreicherungsperiode zwischen 10 und 48 Stunden liegt oder
2) eine Übertragung der Probe in ein wäßriges Wachstumsmedium für Salmonella, wobei das Wachstumsmedium auf einen pH von 5,6 bis 9,3 gepuffert ist, wobei das Wachstumsmedium mit der Probe bei einer Temperatur von 39-42,5°C gehalten wird, wobei Tetrathionat und/oder eine andere selektive Substanz, die nicht Selenit ist, dem Medium vor, gleichzeitig mit oder höchstens 8 Stunden nach der Übertragung hinzugefügt wird oder in diesem erzeugt wird, und wobei die so erhaltene Mischung bei einer Temperatur von 39-42,5°C für eine Zeitspanne von 10-48 Stunden nach der Übertragung (Anreicherungsperiode) gehalten wird,
woraufhin eine Bestimmung zur Identifizierung und/oder Quantifizierung von Salmonella durchgeführt wird, die in dem Anreicherungsmedium nach der Proliferation enthalten sind, wobei die Bestimmung einen Analyseschritt enthält, der auf der Identifikation und/oder Quantifizierung eines Zielmoleküls basiert oder einer molekularen Wechselwirkung, die ein Zielmolekül involviert und die sich von den traditionellen Agarplattierungsverfahren unterscheidet,
wobei die Bedingungen des Anreicherungsverfahrens so an die Bestimmung angepaßt sind, daß ein Test (Test 1),
der das Anreicherungsverfahren und die Bestimmung enthält, worin das selektive Anreicherungsmedium mit einem Stamm von Salmonella zu einer Anfangskonzentration von ungefähr 15 Zellen/ml inokuliert wurde und 10% zufällig ausgewähltes rohes Hackfleisch dem Anreicherungsmedium zu Beginn der Anreicherungsperiode hinzugefügt wurde und die so erhaltene Mischung bei der Temperatur oder den Temperaturen für die Anreicherungsperiode gehalten wurde,
in der Bestimmung ein positives Ergebnis bei mindestens 90% aller Fälle ergeben wird, wenn eine Gruppe von zufällig gewählten Salmonella-Serotypen getestest wird und ein Test (Test 2),
der das Anreicherungsverfahren und die Bestimmung enthält, wobei das selektive Anreicherungsmedium zu Beginn der Anreicherungsperiode mit einem Stamm von einer der Nicht-Salmonella-Arten inokuliert wurde:
Citrobacter freundii,
Escherichia coli und
Enterobacter cloacae,
zu einer Anfangskonzentration von ungefähr 100 Zellen/ml, wobei das selektive Anreicherungsmedium im wesentlichen frei von Mikroorganismen ist, außer dem inokulierten Nicht-Salmonella-Stamm, und wobei die so erhaltene Mischung bei der Temperatur oder den Temperaturen für die Anreicherunsperiode gehalten wird
zu einer Prozentzahl von Falsch-positiven bei der Analyse von höchstens 15% innerhalb jeder Art führen wird, wenn Gruppen von 20 Stämmen getestet werden, die zufällig aus Nicht-Salmonella-Arten ausgewählt wurden.

2. Verfahren gemäß Anspruch 1, wobei die Temperatur während mindestens einem wesentlichen Teil der Anreicherungsperiode im Bereich zwischen 40 und 42°C liegt, vorzugsweise zwischen 40,5 und 41,5°C.

3. Verfahren gemäß Anspruch 1 oder 2, wobei das Tetrathionat und/oder eine andere selektive Substanz, die nicht Selenit ist, dem Medium höchstens 4 Stunden, wie z.B. höchstens 2 Stunden, höchstens 1 Stunde und höchstens 10 Minuten nach der Übertragung hinzugefügt wird oder in diesem erzeugt wird.

4. Verfahren gemäß einem oder mehreren der vorstehenden Ansprüche, Variante 2), wobei die einzige selektive Substanz, die dem Medium vor, gleichzeitig mit oder nach der Übertragung hinzugefügt oder in diesem erzeugt wird, Novobiocin ist.

5. Verfahren gemäß Anspruch 4, wobei die Konzentration von Novobiocin in der erhaltenen Mischung im Bereich von ungefähr 1 bis 100 µg pro ml liegt, wie z.B. im Bereich von ungefähr 1-20 und 2-13 µg pro ml.

6. Verfahren gemäß einem oder mehreren der Ansprüche 1-3, Variante 1), wobei die Dauer der Voranreicherungsperiode im Bereich von ½-7 Stunden liegt, wie z.B. im Bereich von 1-6½ Stunden, 2-6 Stunden und 3-5 Stunden und vorzugsweise bei ungefähr 4 Stunden.

7. Verfahren gemäß einem oder mehreren der vorstehenden Ansprüche, wobei die Summe der Zeit des Anreicherungsverfahrens und des Bestimmungsschritts eine Dauer von höchstens 56 Stunden hat, so wie eine Dauer von höchstens 48 Stunden, 40 Stunden, 34 Stunden, 32 Stunden, 28 Stunden, 24 Stunden, 22 Stunden und 20 Stunden.

8. Verfahren gemäß einem oder mehreren der vorstehenden Ansprüche, wobei die Dauer der Anreicherungsperiode oder in bezug auf Variante 1), die Summe der Voranreicherungsperiode und der Anreicherungsperiode bei 15-30 Stunden liegt, wie z.B. 17-24 Stunden.

9. Verfahren für die Bestimmung von Bakterien, die der Gattung Salmonella angehören, in einer Probe, wobei das Verfahren folgende Schritte umfaßt:
ein Anreicherungsverfahren, das die Übertragung der Probe auf ein wäßriges Wachstumsmedium für Salmonella enthält, wobei das Wachstumsmedium auf einen pH von 5,6 bis 9,3 gepuffert ist, wobei Tetrathionat dem Medium vor, gleichzeitig mit oder höchstens 3 Stunden nach der Übertragung hinzugefügt wird oder in diesem erzeugt wird, und wobei die so erhaltene Mischung bei einer Temperatur von ungefähr 36-39°C für eine Zeitspanne von 15-30 Stunden nach der Übertragung der Probe gehalten wird (Anreicherungsperiode),
und daraufhin Durchführung einer Bestimmung zur Identifizierung und/oder Quantifikation von Salmonella in dem Anreicherungsmedium nach der Proliferation, wobei die Bestimmung einen Analyseschritt enthält, der eine Dauer von höchstens 7 Stunden hat und der auf der Identifizierung und/oder Quantifizierung eines Zielmoleküls beruht oder einer molekularen Interaktion, die ein Zielmolekül involviert und der sich von traditionellen Agarplattierungsverfahren unterscheidet,
wobei die Summe der Zeit des Anreicherungsverfahrens und der Bestimmung eine Dauer von höchstens 40 Stunden hat und die Bedingungen des Anreicherungsverfahrens so an die Bestimmung angepaßt sind, daß Test 1, wie definiert in Anspruch 1, bei der Bestimmung ein positives Ergebnis in mindestens 90% aller Fälle ergeben wird, wenn eine Gruppe von zufällig gewählten Salmonella-Serotypen getestet wird und daß Test 2, wie definiert in Anspruch 1, zu einer Prozentzahl von Falsch-positiven in der Analyse von höchstens 15% innerhalb jeder Art führen wird, wenn eine Gruppe von 20 Stämmen getestet wird, die zufällig ausgewählt wurden aus Nicht-Salmonella-Arten.

10. Verfahren gemäß Anspruch 9, wobei die Summe der Zeit des Anreicherungsverfahrens und des Bestimmungsschritts eine Dauer von höchstens 34 Stunden hat, wie z.B. eine Dauer von höchstens 32 Stunden, 28 Stunden, 24 Stunden, 22 Stunden und 20 Stunden.

11. Verfahren gemäß Anspruch 9 oder 10, wobei die Dauer der Anreicherungsperiode 17-24 Stunden beträgt.

12. Verfahren gemäß einem oder mehreren der Ansprüche 9-11, wobei die Temperatur im Bereich von 36°C und 39°C während mindestens eines wesentlichen Teils der Anreicherungsperiode liegt.

13. Verfahren gemäß einem oder mehreren der Ansprüche 9-12, wobei das Tetrathionat dem Medium höchstens 1 Stunde nach der Übertragung hinzugefügt wird oder in diesem erzeugt wird, wie z.B. höchstens 5 Minuten.

14. Verfahren gemäß einem oder mehreren der vorstehenden Ansprüche, das gegen das Standardprotokoll NMKL Nr. 71 (Nordic Committee on Food Analysis method Nr. 71, 1991, fourth edition, seventeenth printing) kalibriert ist, mit den Ausnahmen, daß Oxoid CM 866 Rappaport-Vassiliadis broth (RVS) anstelle von Merck 7700 Rappaport-Vassiliadis broth (RV) verwendet wird und daß RVS bei 42°C inkubiert wird und daß Gibco 152-05600/Oxoid CM 469 Xylose Lysin-Desoxycholatagar (XLD) ebenfalls als Plattierungsmedium verwendet wird in einer Weise, daß die Bestimmung zu mindestens 80% richtig positiven Ergebnissen führt, verglichen mit dem Standardprotokoll, und höchstens 10% falschpositive Resultate ergibt, wenn 100 Proben von Hackfleisch (Test 3) getestet werden, und wobei das Gewicht des Teils der Probe, der durch jedes der zwei Verfahren getestet werden soll, 25 g beträgt, wobei die 100 Proben von 10 verschiedenen Kleinverkäufen (retails) zur Verfügung gestellt werden, wobei jeder 10 verschiedene Proben liefert, wobei 20 der zur Verfügung gestellten Proben mit ungefähr 15 Salmonella typhimurium-Zellen pro Probe inokuliert wurden und 20 der zur Verfügung gestellten Proben mit ungefähr 15 Salmonella enteritidis-Zellen pro Probe inokuliert wurden.

15. Verfahren gemäß einem oder mehreren der vorstehenden Ansprüche, wobei die Probe sich von einem verarbeiteten Produkt unterscheidet.

16. Verfahren gemäß Anspruch 14 oder 15, wobei die Bestimmung zu mindestens 85% richtig positiven Ergebnissen führt, verglichen mit dem Standardprotokoll in Test 3, wie z.B. mindestens 90%, 92%, 95%, 97%, 98%, 99% und 100% richtig positive Ergebnisse.

17. Verfahren gemäß Anspruch 16, wobei die Bestimmung zu mindestens 101% richtig positiven Ergebnissen führt, wenn verglichen mit dem Standardprotokoll in Test 3.

18. Verfahren gemäß einem oder mehreren der Ansprüche 14-17, wobei die Bestimmung zu höchstens 8% falsch-positiven Ergebnissen in Test 3 führt, wie z.B. höchstens 5%, 3%, 2% und 1% falsch-positiven Ergebnissen.

19. Verfahren gemäß Anspruch 18, wobei die Bestimmung zu 0 falsch-positiven Ergebnissen in Test 3 führt.

20. Verfahren gemäß einem oder mehreren der vorstehenden Ansprüche, wobei die Bestimmung zu mindestens 92% richtig-positiven Ergebnissen in allen Fällen des Tests 1 führt, wie z.B. mindestens 95%, 97%, 98%, 99%, 99,5% und 99,9% richtig positiven Ergebnissen.

21. Verfahren gemäß einem oder mehreren der vorstehenden Ansprüche, wobei die Bestimmung zu höchstens 12% falsch-positiven Ergebnissen in Test 2 führt, wie z.B. höchstens 10%, 7%, 5%, 3%, 2%, 1% und ½% falsch-positiven Ergebnissen.

22. Verfahren gemäß einem oder mehreren der vorstehenden Ansprüche, wobei die Konzentration des Tetrathionats in der erhaltenen Mischung im Bereich von ungefähr 5-40 mM liegt, wie z.B. im Bereich von ungefähr 7-35 mM, 9-30 mM, 11-28 mM, 13-26 mM, 14-25 mM, 15-23 mM, 16-22 mM, 17-21 mM und 18-20 mM.

23. Verfahren für die Bestimmung von Bakterien in einer Probe, die der Gattung Salmonella angehören,
wobei das Verfahren die Durchführung eines Anreicherungsverfahrens enthält, das entweder folgenden Schritt enthält
a) Übertragung der Probe auf ein wäßriges Wachstumsmedium für Salmonella, wobei das Wachstumsmedium auf einen pH zwischen 5,6 und 9,3 gepuffert ist und im wesentlichen keine Substanz enthält, die das bakterielle Wachstum inhibiert und wobei die so erhaltene Mischung bei einer Temperatur von höchstens 39°C für eine Zeitspanne von mindestens 1 Minute (Voranreicherungsperiode) gehalten wird, woraufhin
die Temperatur der Mischung auf 39,5-43°C erhöht wird und gegebenenfalls Novobiocin und/oder eine andere selektive Substanz, die nicht Selenit ist, zugefügt wird,
und woraufhin die Mischung bei der erhöhten Temperatur von 39,5-43°C für eine Zeitspanne ab dem Temperaturanstieg (selektive Anreicherungsperiode) gehalten wird oder
b) Übertragung der Probe auf ein wäßriges Wachstumsmedium für Salmonella, wobei das Wachstumsmedium auf einen pH zwischen 5,6 und 9,3 gepuffert ist, wobeiNovobiocin und/oder eine andere selektive Substanz, die nicht Selenit ist, dem Medium vor, gleichzeitig mit oder nach der Übertragung hinzugefügt wird und wobei die erhaltene Mischung bei einer Temperatur von höchstens 39°C für eine Zeitspanne von mindestens einer halben Stunde nach der Übertragung oder der Hinzugabe des Novobiocins oder einer anderen selektiven Substanz, die nicht Selenit ist, gehalten wird, je nachdem welches der spätere Zeitpunkt ist (Voranreicherungsperiode) und woraufhin
die Temperatur der Mischung auf 39,5-43°C erhöht wird, und gegebenenfalls Novobiocin oder eine andere selektive Substanz, die nicht Selenit ist, hinzugefügt wird, und die Mischung bei der erhöhten Temperatur von 39,5-43°C für eine Zeitspanne ab dem Temperaturanstieg (selektive Anreicherungsperiode) gehalten wird,
woraufhin eine Bestimmung zur Identifizierung und/oder Quantifikation von Salmonella durchgeführt wird, die im Anreicherungsmedium nach der Proliferation enthalten ist, wobei die Summe der Zeitspannen der Voranreicherungsperiode und der selektiven Anreicherungsperiode zwischen 10-48 Stunden liegt.

24. Verfahren gemäß Anspruch 23, wobei die Dauer der Voranreicherungsperiode mindestens eine halbe Stunde beträgt, wie z.B. 1-10 Stunden, 1-8 Stunden, 2-6 Stunden, 3-5 Stunden und 3½-4½ Stunden und wobei die Dauer vorzugsweise ungefähr 4 Stunden beträgt.

25. Verfahren gemäß Anspruch 23 oder 24, wobei die Summe der Zeitspanne des Anreicherungsverfahrens und des Bestimmungsschritts eine Dauer von höchstens 56 Stunden hat, wie z.B. eine Dauer von höchstens 48 Stunden, 40 Stunden, 32 Stunden, 28 Stunden, 24 Stunden, 22 Stunden und 20 Stunden.

26. Verfahren gemäß einem oder mehreren der Ansprüche 23-25, wobei die Dauer der Summe der Voranreicherungsperiode und der selektiven Anreicherungsperiode 15-30 Stunden beträgt, wie z.B. 17-24 Stunden.

27. Verfahren für die Bestimmung von Bakterien, die der Gattung Salmonella angehören, in einer Probe, wobei das Verfahren den Schritt der Durchführung eines Anreicherungsverfahrens enthält, das folgende Schritte umfaßt:
Übertragung der Probe auf ein wäßriges Wachstumsmedium für Salmonella, wobei das Wachstumsmedium auf einen pH zwischen 5,6 und 9,3 gepuffert ist, wobei Novobiocin dem Medium vor, gleichzeitig mit oder nach der Übertragung hinzugefügt wird und wobei die erhaltene Mischung bei einer Temperatur von höchsens 39°C für eine Zeitspanne von 15-30 Stunden nach der Übertragung gehalten wird, oder nach der Zufuhr des Novobiocins oder einer anderen möglichen selektiven Substanz, die nicht Selenit ist, je nachdem welches der spätere Zeitpunkt ist (Anreicherungsperiode),
und daraufhin eine Durchführung einer Bestimmung, die eine Dauer von höchstens 7 Stunden hat, zur Identifizierung und/oder Quantifikation von Salmonella, die im Anreicherungsmedium nach Proliferation vorhanden ist,
wobei die Summe der Zeit des Anreicherungsverfahrens und der Bestimmung eine Dauer von höchstens 40 Stunden hat.

28. Verfahren gemäß Anspruch 27, wobei die Summe der Zeit des Anreicherungsverfahrens und des Bestimmungsschritts eine Dauer von höchstens 32 Stunden hat, wie z.B. eine Dauer von höchstens 28 Stunden, 24 Stunden, 22 Stunden und 20 Stunden.

29. Verfahren gemäß Anspruch 27 oder 28, wobei die Dauer der Anreicherungsperiode 17-24 Stunden beträgt.

30. Verfahren gemäß einem oder mehreren der Ansprüche 23-29, wobei die Bestimmung einen Analyseschritt enthält, der auf der Identifizierung und/oder Quantifikation eines Zielmoleküls basiert oder einer molekularen Interaktion, die ein Zielmolekül involviert und wobei der Analyseschritt sich von der Agarplattierung unterscheidet.

31. Verfahren gemäß einem oder mehreren der Ansprüche 23-30, wobei die Bedingungen des Anreicherungsverfahrens so auf die Bestimmung angepaßt sind, daß
- ein Test (Test 4), der das Anreicherungsverfahren und die Bestimmung enthält, wobei das wäßrige Wachstumsmedium mit hitze-behandelten Zellen eines Stammes von Salmonella zu einer Anfangskonzentration von ungefähr 1000 hitze-behandelten Zellen pro ml inokuliert wurde und 5% nicht-fette Trockenmilch dem Medium zu Beginn des Anreicherungsverfahrens hinzugefügt wurde, bei der Bestimmung ein positives Ergebnis in 90% aller Fälle ergeben wird, wenn eine Gruppe von zufällig gewählten Salmonella-Serotypen getestet wird und
- ein Test (Test 5), der das Anreicherungsverfahren und die Bestimmung enthält, wobei das wäßrige Wachstumsmedium mit einem Stamm von einer der Nicht-Salmonella-Arten
Citrobacter freundii,
Escherichia coli und
Enterobacter cloacae,
inokuliert wurde zu einer Anfangskonzentration von ungefähr 100 Zellen/ml zu Beginn des Anreicherungsverfahrens, zu einer Prozentzahl von Falsch-positiven bei der Bestimmung von höchstens 25% innerhalb jeder Art einer Gruppe von 20 zufällig ausgewählten Arten der Nicht-Salmonella-Stämme führen wird.

32. Verfahren gemäß einem oder mehreren der Ansprüche 23-31, wobei es gegen das Standardkulturprotokoll NMKL Nr. 71 (Nordic Committee on Food Analysis method Nr. 71, 1991, fourth edition, seventeenth printing) derartig kalibriert ist, daß die Bestimmung zu mindestens 80% richtig-positiven Ergebnissen führt, verglichen mit dem Standardprotokoll, und zu höchstens 10% falsch-positiven Ergebnissen führt, wenn eine Gesamtmenge von 50 Proben aus 50 g Milchpulver getestet wird (Test 6), zufällig gewählt in bezug auf Charge und Art, wobei jede Probe eine Abteilung von zwei Teilen zu 25 g unterteilt ist, wobei ein Teil jeder Abteilung als Probe dem Verfahren gemäß einem oder mehreren der Ansprüche 23-31 unterzogen wird und der andere Teil von jeder Abteilung als Probe dem Standardkulturprotokoll unterzogen wird, wobei 25 der 50 Abteilungen von 25 g Teilen in der folgenden Weise mit Salmonella versehen wird:
- ein 25 g Teil der Abteilung, der dem Standardkulturprotokoll unterzogen wird, wird durch Zugabe zu Beginn des Anreicherungsverfahrens des Protokolls von 0,1-1 g nicht-fetter Trockenmilch, die ungefähr 5 CFU Salmonella panama oder Salmonella typhimurium enthält, zu dem wäßrigen Wachstumsmedium, das in dem Protokoll verwendet wird, supplementiert,
- der andere 25 g Teil der Abteilung, der dem Verfahren gemäß einem oder mehreren der Ansprüche 23-31 unterzogen wird, wird durch Zugabe zu Beginn des Anreicherungsverfahrens von 0,1-1 g nicht-fetter Trockenmilch, die ungefähr 5 CFU Salmonella panama oder Salmonella typhimurium enthält, zu dem wäßrigen Wachstumsmedium supplementiert.

33. Verfahren gemäß Anspruch 32, wobei die Bestimmung zu mindestens 85% richtig positiven Ergebnissen führt, verglichen mit dem Standardprotokoll in Test 6, wie z.B. mindestens 90%, 92%, 95%, 97%, 98%, 99% und 100% richtig positiven Ergebnissen.

34. Verfahren gemäß Anspruch 33, wobei die Bestimmung zu mindestens 101% richtig positiven Ergebnissen, verglichen mit dem Standardprotokoll in Test 6 führt.

35. Verfahren gemäß einem oder mehreren der Ansprüche 32-34, wobei die Bestimmung zu höchstens 8% falsch-positiven Ergebnissen in Test 6 führt, wie z.B. höchstens 5%, 3%, 2% und 1% falsch-positiven Ergebnissen.

36. Verfahren gemäß Anspruch 35, wobei die Bestimmung zu 0 falsch-positiven Ergebnissen in Test 6 führt.

37. Verfahren gemäß einem oder mehreren der Ansprüche 23-36, wobei die Bestimmung zu mindestens 92% richtig-positiven Ergebnissen in allen Fällen von Test 4 führt, wie mindestens 95%, 97%, 98%, 99%, 99,5% und 99,9% richtig-positiven Ergebnissen.

38. Verfahren gemäß einem oder mehreren der Ansprüche 23-37, wobei die Bestimmung zu höchstens 20% falsch-positiven Ergebnissen in Test 5 führt, wie z.B. höchstens 15%, 10%, 6%, 3%, 2%, 1% und ½%.

39. Verfahren gemäß einem oder mehreren der vorstehenden Ansprüche, wobei die Probe von einem Tier genommen wird, einschließlich einem Menschen.

40. Verfahren gemäß Anspruch 39, wobei der Ursprung der Probe aus der Gruppe gewählt ist, die besteht aus Fäkalien, Magensaft, Urin, Blut, Lymphflüssigkeit, Cerebrospinalflüssigkeit und einer Biopsie.

41. Verfahren gemäß einem oder mehreren der Ansprüche 1-38, wobei die Probe ihren Ursprung in der Umwelt hat.

42. Verfahren gemäß Anspruch 41, wobei die Probe ausgewählt ist aus der Gruppe bestehend aus Staub, Abstrichen (Swabs) und Flaum.

43. Verfahren gemäß einem oder mehreren der Ansprüche 23-38, wobei die Probe ein verarbeitetes Produkt ist.

44. Verfahren gemäß einem oder mehreren der Ansprüche 1-38 und 43, wobei die Probe von einem Nahrungsmittel oder Futtermittel genommen wird.

45. Verfahren gemäß einem oder mehreren der vorstehenden Ansprüche, wobei der Bestimmungsschritt eine immunologische Reaktion enthält.

46. Verfahren gemäß Anspruch 45, wobei der Bestimmungsschritt eine Analyse enthält, die aus der Gruppe ausgewählt ist, bestehend aus
einem Immun-Assay, wie z.B. einem Radio-Immunassay (RIA), einem Enzym Immunassay (EIA), einem Enzyme-Linked-Immuno-Sorbent-Assay (ELISA), einer Fluoreszenz-Antikörpertechnik (FA) und einem Immunassay, der die Verwendung von Liposomen, Micellen oder liposomen-ähnlichen Partikeln in der Detektionsphase enthält;
einem Assay, der eine Immunimmobilisierung involviert;
einem Assay, der eine Immunagglutination, so wie eine Latexagglutination, enthält;
einem DNA/DNA-Hybridisierungsassay;
einem RNA/RNA-Hybridisierungsassay; und
einem DNA/RNA-Hybridisierungsassay.

47. Verfahren gemäß einem oder mehreren der Ansprüche 1-44, wobei der Bestimmungsschritt eine Polymerase-Kettenreaktion umfaßt (PCR).

48. Verfahren gemäß einem oder mehreren der Ansprüche 1-44, wobei der Bestimmungsschritt die Verwendung von Peptidnukleinsäuren enthält (PNA).

49. Verfahren gemäß einem oder mehreren der Ansprüche 1-44, wobei der Bestimmungsschritt die Verwendung von Lektinen umfaßt.

50. Verfahren gemäß einem oder mehreren der Ansprüche 1-44, wobei der Bestimmungsschritt die Verwendung von Bakteriophagen, wie z.B. dem Bakteriophagen Felix 01, umfaßt.

51. Verfahren gemäß einem oder mehreren der Ansprüche 1-44, wobei der Bestimmungsschritt eine metabolische Umwandlung einer Substanz durch Salmonella enthält, was zu einer Veränderung des elektrischen Widerstands oder der Impedanz des Mediums, das Salmonella enthält, führt, und wobei die Detektion von Salmonella durch eine Messung dieser Veränderung erreicht wird.

52. Verfahren gemäß einem oder mehreren der Ansprüche 1-44, wobei der Bestimmungsschritt einen Assay für die Fähigkeit von Salmonella zur Wanderung auf oder durch eine Agarmatrix enthält.

53. Verfahren gemäß einem oder mehreren der Ansprüche 1-44, wobei der Bestimmungsschritt einen Assay enthält, der einen Filtrationsschritt aufweist, wobei die Bakterien von dem Medium getrennt und auf einem Filter zurückgehalten werden, wie z.B. einen Assay, der ein hydrophobes Gittermembran-Filterverfahren (HGMF) enthält.

54. Verfahren gemäß einem oder mehreren der Ansprüche 1-44, wobei der Bestimmungsschritt einen Assay enthält, der ein radiometrisches Verfahren enthält.

55. Verfahren gemäß einem oder mehreren der Ansprüche 1-44, wobei der Bestimmungsschritt einen Assay enthält, der eine chromatographische Trennung enthält.

56. Verfahren gemäß einem oder mehreren der vorstehenden Ansprüche, wobei der Bestimmungsschritt eine selektive Nachanreicherung umfaßt.

57. Verfahren gemäß Anspruch 56, wobei die selektive Nachanreicherung eine Dauer von 2-6 Stunden aufweist, wie z.B. eine Dauer von 2,0-4,5 Stunden, 2,5-3,5 Stunden und vorzugsweise ungefähr 3 Stunden.

58. Verfahren gemäß einem oder mehreren der vorstehenden Ansprüche, wobei das wässerige Wachstumsmedium für Salmonella eine Konzentration an Selenit aufweist, die höchstens der Selenitkonzentration in einer 0,5%igen w/v Natriumhydrogenselenitlösung entspricht, vorzugsweise eine Selenitkonzentration von 0% w/v.

## Revendications

1. Procédé de détermination de bactéries appartenant au genre *Salmonella* dans un échantillon,
le procédé comprenant un procédé d'enrichissement dans lequel ou bien
1) on transfère l'échantillon dans un milieu de croissance aqueux pour *Salmonella,* le milieu de croissance étant tamponné à un pH compris entre 5,6 et 9,3, avec addition ou production dans le milieu avant, en même temps que ou après le transfert, de tétrathionate et/ou d'une autre substance sélective différente de la sélénite, et maintien du mélange résultant à une température d'au plus 38°C pendant une durée comprise entre 10 minutes et 8 heures après le transfert de l'échantillon (période de pré-enrichissement) puis accroissement de la température du mélange à 39-43°C et maintien du mélange à la température augmentée de 39-43°C pendant une certaine durée après l'accroissement de la température (période d'enrichissement), la somme des durées de la période de pré-enrichissement et de la période d'enrichissement étant comprise entre 10 et 48, ou bien
2) on transfère l'échantillon dans un milieu de croissance aqueux pour *Salmonella,* le milieu de croissance étant tamponné à un pH compris entre 5,6 et 9,3, le milieu de croissance avec l'échantillon étant maintenu à une température de 39-42,5°C, avec addition ou production de tétrathionate et/ou d'une autre substance sélective différente de la sélénite dans le milieu avant, en même temps que ou au plus 8 heures après le transfert et maintien du mélange résultant à une température de 39-42,5°C pendant une durée comprise entre 10 et 48 heures à partir du transfert (période d'enrichissement),
puis on réalise une détermination afin d'identifier et/ou de quantifier les *Salmonella* présents dans le milieu d'enrichissement après la prolifération, la détermination comprenant une étape de test qui se fonde sur l'identification et/ou la quantification d'une molécule cible ou d'une interaction moléculaire faisant intervenir une molécule cible et qui diffère des techniques traditionnelles de dépôt sur gélose,
les conditions d'enrichissement du procédé étant adaptées à la détermination de manière qu'un test (test 1)
comprenant le procédé d'enrichissement et la détermination, où le milieu d'enrichissement sélectif a reçu une inoculation d'une souche de *Salmonella* en une concentration de départ d'environ 15 cellules/ml et où 10% de viande crue hachée sélectionnée au hasard a été ajoutée au milieu d'enrichissement au début de la période d'enrichissement et le mélange résultant a été maintenu à la température ou aux températures de la période d'enrichissement produise, dans la détermination, un résultat positif dans au moins 90% de tous les cas lorsqu'on teste un échantillon de sérotypes de *Salmonella* choisi au hasard, et qu'un test (test 2)
comprenant le procédé d'enrichissement et la détermination, où le milieu d'enrichissement sélectif a reçu une innoculation au début de la période d'enrichissement avec une souche d'une des espèces non-*Salmonella*
*Citrobacter freundii*
*Escherichia coli,* et
*Enterobacter clocae*
en une concentration de départ d'environ 100 cellules/ml, le milieu d'enrichissement sélectif étant sensiblement dépourvu de micro-organismes autres que la souche non-*Salmonella* inoculée, et le mélange résultant ayant été maintenu à la température ou aux températures nécessaires pour la période d'enrichissement.
donne naissance à un pourcentage de résultats faussement positifs dans le test d'au plus 15% dans chaque espèce lorsqu'on teste un échantillon de 20 souches choisies au hasard dans les espèces non-*Salmonella.*

2. Procédé selon la revendication 1, dans lequel la température est située dans un intervalle compris entre 40 et 42°C, de préférence entre 40,5 et 41,5°C, pendant au moins une partie substantielle de la période d'enrichissement.

3. Procédé selon la revendication 1 ou 2, dans lequel on ajoute ou on produit dans le milieu le tétrathionate et/ou une autre substance sélective différente de la sélénite au plus 4 heures, comme au plus 2 heures, au plus 1 heure et au plus 10 minutes après le transfert.

4. Procédé selon l'une quelconque des revendications précédentes, variante 2), dans lequel la seule substance sélective qui est ajoutée ou produite dans le milieu, avant, en même temps que ou après le transfert est la novobiocine.

5. Procédé selon la revendication 4, dans lequel la concentration de novobiocine dans le mélange résultant est dans un intervalle d'environ 1 à 100 µg/ml, comme dans un intervalle d'environ 1 à 20 et 2 à 13 µg/ml.

6. Procédé selon l'une quelconque des revendications 1-3, variante 1), dans lequel la durée de la période de pré-enrichissement est comprise dans un intervalle allant de 1/2 heure à 7 heures, comme dans un intervalle allant de 1 heure à 6 heures 1/2, de 2 à 6 heures, et de 3 à 5 heures, et de préférence la durée est d'environ 4 heures.

7. Procédé selon l'une quelconque des revendications précédentes, dans lequel la somme de la durée du procédé d'enrichissement et de l'étape de détermination a une durée d'au plus 56 heures, comme une durée d'au plus 48 heures, 40 heures, 34 heures, 32 heures, 28 heures, 24 heures, 22 heures, et 20 heures.

8. Procédé selon l'une quelconque des revendications précédentes, dans lequel la durée de la période d'enrichissement ou, en ce qui concerne la variante 1) la somme de la période de pré-enrichissement et de la période d'enrichissement, est de 15-30, comme de 17-24 heures.

9. Procédé pour la détermination de bactéries appartenant au genre *Salmonella* dans l'échantillon, le procédé comprenant
un procédé d'enrichissement dans lequel on transfère l'échantillon dans un milieu de croissance aqueux pour *Salmonella,* le milieu de croissance étant tamponné à pH compris entre 5,6 et 9,3, avec addition ou production de tétrathionate dans le milieu avant, en même temps que ou au plus trois heures après le transfert, et on maintient le mélange résultant à une température d'environ 36-35°C pendant une durée comprise entre 15 et 30 heures après le transfert de l'échantillon (période d'enrichissement),
puis on réalise une détermination afin d'identifier et/ou de quantifier les *Salmonella* présents dans le milieu d'enrichissement après la prolifération, la détermination comprenant une étape de test qui a une durée d'au plus 7 heures et qui se fonde sur l'identification et/ou la quantification d'une molécule cible ou une interaction moléculaire faisant intervenir une molécule cible et qui diffère des techniques traditionnelles de dépôt sur gélose,
la somme de la durée du procédé d'enrichissement et de la détermination ayant une durée d'au plus 40 heures et les conditions du procédé d'enrichissement étant adaptées à la détermination de manière que le test 1 tel que défini dans la revendication 1 produise, dans la détermination, un résultat positif dans au moins 90% de tous les cas, lorsqu'on teste un échantillon de sérotypes de *Salmonella* choisis au hasard, et que le test 2 tel que défini dans la revendication 1 donne naissance à un pourcentage de résultats faussement positifs dans le test d'au plus 15% dans chaque espèce lorsqu'on teste un échantillon de 20 souches choisies au hasard parmi les espèces non-*Salmonella.*

10. Procédé selon la revendication 9, dans lequel la somme de la durée du procédé d'enrichissement et de l'étape de détermination a une durée d'au plus 34 heures, comme une durée d'au plus 32 heures, 28 heures, 24 heures, 22 heures, et 20 heures.

11. Procédé selon la revendication 9 ou 10, dans lequel la durée de la période d'enrichissement est de 17-24 heures.

12. Procédé selon l'une quelconque des revendications 9-11, dans lequel la température est dans un intervalle compris entre 36°C et 39°C pendant au moins une partie substantielle de la période d'enrichissement.

13. Procédé selon l'une quelconque des revendications 9-12, dans lequel on ajoute ou on produit du tétrathionate dans le milieu au plus une heure après le transfert, comme au bout d'au plus 5 minutes.

14. Procédé selon l'une quelconque des revendications précédentes, qui est étalonné par rapport au protocole de référence NMKL N°71 (procédé Nordic Committee on Food Analysis N° 71, 1991, 4ème édition, 17ème réimpression), avec ces exceptions que l'on utilise le bouillon Rappaport-Vassiliadis (RVS) d'Oxoïd CM 866 au lieu du bouillon Rappaport-Vassiliadis (RV) de Merck 7700 et qu'on fait incuber le RVS à 42°C, et qu'on utliise également une gélose de désoxycholate de xylose-lysine (XLD) de Gibco 152-05600/Oxoïd CM 469 comme milieu de dépôt, de manière telle que la détermination aboutisse à au moins 80% de résultats positifs vrais par comparaison avec le protocole de référence et donne naissance à au plus 10% de résultats positifs faux lorsqu'on teste 100 échantillons de viande hachée (test 3), et que le poids de la partie de l'échantillon à tester par chacun des deux procédés est de 25 grammes, les 100 échantillons provenant de 10 magasins de détail différents, dont chacun délivre 10 échantillons différents, 20 des échantillons founis ayant reçu une inoculation d'environ 15 cellules de *Salmonella typhimurium* par échantillon et 20 des échantillons fournis ayant reçu une inoculation d'environ 15 cellules de *Salmonella* enterititis par échantillon.

15. Procédé selon l'une quelconque des revendications précédentes, dans lequel l'échantillon diffère d'un produit traité.

16. Procédé selon la revendication 14 ou 15, dans lequel la détermination donne naissance à au moins 85% de résultats positifs vrais par comparaison avec le protocole de référence dans le test 3, comme au moins 90%, 92%, 95%, 97%, 98%, 99% et 100% de résultats positifs vrais.

17. Procédé selon la revendication 16, dans lequel la détermination donne naissance à au moins 101% de résultats positifs vrais par comparaison avec le protocole de référence dans le test 3.

18. Procédé selon l'une quelconque des revendications 14-17, dans lequel la détermination donne naissance à au plus 8% de résultats positifs faux dans le test 3, comme au plus 5%, 3%, 2%, et 1% de résultats positifs faux.

19. Procédé selon la revendication 18, dans lequel la détermination donne naissance à 0 résultat positif faux dans le test 3.

20. Procédé selon l'une quelconque des revendications précédentes, dans lequel la détermination donne naissance à au moins 92% de résultats positifs vrais dans tous les cas du test 1, comme au moins 95%, 97%, 98%, 99%, 99,5% et 99,9% de résultats positifs vrais.

21. Procédé selon l'une quelconque des revendications précédentes, dans lequel la détermination donne naissance à au plus 12% de résultats positifs faux dans le test 2, comme au plus 10%, 7%, 5%, 3%, 2%, 1%, et 1/2% de résultats positifs faux.

22. Procédé selon l'une quelconque des revendications précédentes, dans lequel la concentration de tétrathionate dans le mélange résultant est dans un intervalle de 5-40 mM, comme dans un intervalle d'environ 7-35 mM, 9-30 mM, 11-28 mM, 13-26 mM, 14-25 mM, 15-23 mM, 16-22 mM, 17-21 mM et 18-20 mM.

23. Procédé de détermination de bactéries appartenant au genre *Salmonella* dans un échantillon,
dans lequel on réalise un procédé d'enrichissement qui comprend ou bien
a) le transfert de l'échantillon dans un milieu de croissance aqueux pour *Salmonella,* le milieu de croissance étant tamponné à un pH compris entre 5,6 et 9,3, et ne contenant sensiblement pas de substance inhibant la croissance bactérienne, et le maintien du mélange résultant à une température d'au plus 39,0°C pendant une durée d'au moins d'une minute, (période de pré-enrichissement), puis
l'accroissement de la température à 39,5-43°C, et facultativement l'addition de novobiocine et/ou d'une autre substance sélective différente de la sélénite
et le maintien du mélange à la température augmentée de 39,5-43°C pendant une durée commençant avec l'augmentation de température (période d'enrichissement sélectif), ou bien
b) le transfert de l'échantillon dans un milieu de croisance aqueux pour *Salmonella,* le milieu de croissance étant tamponné à un pH compris entre 5,6 et 9,3, avec addition de novobiocine et/ou d'une autre substance sélective différente de la sélénite au milieu, avant, en même temps que ou après le transfert et le maintien du mélange résultant à une température d'au plus 39,0°C pendant une durée d'au moins une demi-heure à partir du transfert ou de l'addition de novobiocine ou d'une autre substance sélective différant de la sélénite, quelle que soit cette dernière (période de pré-enrichissement),
puis l'augmentation de la température du mélange à 39,5-43°C et facultativement l'addition de novobiocine ou une d'autre substance sélective différente de la sélénite,
et le maintien du mélange à la température augmentée de 39,5-43°C pendant une durée partant de l'augmentation de température (période d'enrichissement sélectif),
puis la réalisation d'une détermination afin d'identifier et/ou de quantifier les *Salmonella* présents dans le milieu d'enrichissement après la prolifération, la somme des durées de la période de pré-enrichissement et de la période d'enrichissement sélectif étant comprise entre environ 10 et 48 heures.

24. Procédé selon la revendication 23, dans lequel la durée de la période de pré-enrichissement est d'au moins 1/2 heure, comme de 1-10 heures, 1-8 heures, 2-6 heures, 3-5 heures et 3 h 1/2-4 h 1/2, et de préférence la durée est d'environ 4 heures.

25. Procédé selon la revendication 23 ou 24, dans lequel la somme de la durée du procédé d'enrichissement et de l'étape de détermination a une durée d'au plus 56 heures, comme une durée d'au plus 48 heures, 40 heures, 32 heures, 28 heures, 24 heures, 22 heures et 20 heures.

26. Procédé selon l'une quelconque des revendications 23-25 dans lequel la somme de la durée de la période de pré-enrichissement et de la durée de la période d'enrichissement sélectif est de 15-30 heures, comme 17-24 heures.

27. Procédé de détermination de bactéries appartenant au genre *Salmonella* dans un échantillon, dans lequel on réalise une opération d'enrichissement comprenant
le transfert de l'échantillon dans un milieu de croissance aqueux pour *Salmonella*, le milieu de croissance étant tamponné à un pH compris entre 5,6 et 9,3, avec addition de novobiocine au milieu, avant, en même temps que ou après le transfert, et
le maintien du mélange résultant à une température d'au plus 39,0°C pendant une durée comprise entre 15 et 30 heures à partir du transfert, l'addition de novobiocine ou d'une autre substance sélective facultative différente de la sélénite, quelle que soit cette dernière, (période d'enrichissement), puis la réalisation d'une détermination qui a une durée d'au plus 7 heures pour identifier et/ou quantifier les *Salmonella* présentes dans le milieu d'enrichissement après la prolifération,
la somme de la durée du procédé d'enrichissement et de la détermination ayant une durée d'au plus 40 heures.

28. Procédé selon la revendication 27, dans lequel la somme de la durée du procédé d'enrichissement et de l'étape de détermination a une durée d'au plus 32 heures, comme une durée d'au plus 28 heures, 24 heures, 22 heures, et 20 heures.

29. Procédé selon la revendication 27 ou 28, dans lequel la durée de la période d'enrichissement est de 17 à 24 heures.

30. Procédé selon l'une quelconque des revendications 23 à 29, dans lequel la détermination comprend une étape d'essai qui se fonde sur l'identification et/ou la quantification d'une molécule cible ou d'une interaction moléculaire faisant intervenir une molécule cible, cette étape d'essai différant d'un dépôt sur gélose.

31. Procédé selon l'une quelconque des revendications 23 à 30, dans lequel les conditions du procédé d'enrichissement sont adaptées à la détermination de manière que
- un test (test 4) comprenant le procédé d'enrichissement et la détermination, dans lequel le milieu de croissance aqueux a reçu une inoculation avec des cellules traitées à la chaleur d'une souche de *Salmonella* en une concentration de départ d'environ 1000 cellules traitées à la chaleur par ml, avec addition de 5% de lait concentré à faible teneur en matière grasse au milieu du début du procédé d'enrichissement, produise, dans la détermination, un résultat positif dans 90% de tous les cas, lorsqu'on teste un échantillon de sérotypes de *Salmonella* choisi au hasard, et
- un test (test 5) comprenant le procédé d'enrichissement et la détermination, dans lequel le milieu de croissance aqueux a reçu une inoculation avec une souche d'une des espèces non salmonella.
*Citrobacter freundii,*
*Escherichia coli,* et
*Enterobacter cloacae,*
en une concentration de départ de ces bactéries d'environ 100 cellules/ml au début du procédé d'enrichissement, donne naissance à un pourcentage de résultats faussement positifs dans la détermination d'au plus 25% de chaque espèce d'un échantillon de 20 espèces de souches non Salmonella choisies au hasard.

32. Procédé selon l'une quelconque des revendications 23-31, qui est étalonné par rapport au protocole de culture de référence NMKL N°71 (procédé Nordic Committee on Food Analysis N° 71, 1991, 4ème édition, 17ème réimpression), de manière que la détermination aboutisse à au moins 80% de résultats positifs vrais par comparaison avec le protocole de référence, et donne naissance à au plus 10% de résultats faussement positifs lorsqu'on teste (test 6) un total de 50 échantillons de 50 grammes de lait en poudre choisis au hasard quant au lot et au type, chaque échatillon étant divisé en un ensemble de deux parties de 25 grammes, une partie de chaque ensemble étant soumise en tant qu'échantillon au procédé selon l'une quelconque des revendications 23-31 et l'autre partie de chaque ensemble étant soumise en tant qu'échantillon au protocole de culture de référence, 25 des 50 ensembles de parties de 25 grammes recevant une inoculation de la façon suivante :
- une partie de 25 g de l'ensemble, soumise au protocole de culture de référence, reçoit un supplément par addition, au début du procédé d'enrichissement du protocole, de 0,1-1 g de lait en poudre dépourvu de matières grasses contenant environ 5 CFU de *Salmonella panama* ou *Salmonella typhimurium* au milieu de croissance aqueux utilisé dans le protocole,
- l'autre partie de 25 g de l'ensemble, soumise au procédé selon l'une quelconque des revendications 23-31, reçoit un supplément par addition, au début du procédé d'enrichissement, de 0,1-1 g de lait en poudre dépourvu de matières grasses contenant environ 5 CFU de *Salmonella panama* ou de *Salmonella typhurium* au milieu de croissance aqueux.

33. Procédé selon la revendication 32, dans lequel la détermination donne naissance à au moins 85% de résultats positifs vrais, par comparaison avec le protocole standard dans le test 6, comme au moins 90%, 92%, 95%, 97%, 98%, 99%, et 100% de résultats positifs vrais.

34. Procédé selon la revendication 33, dans lequel la détermination donne naissance à au moins 101% de résultats positifs vrais par comparaison avec le protocole de référence dans le test 6.

35. Procédé selon l'une quelconque des revendications 32-34, dans lequel la détermination donne naissance à au plus 8% de résultats positifs faux dans le test 6, comme au plus 5%, 3%, 2%, et 1% de résultats positifs faux.

36. Procédé selon la revendication 35, dans lequel la détermination donne naissance à 0 résultats positifs faux dans le test 6.

37. Procédé selon l'une quelconque des revendications 23-36, dans lequel la détermination donne naissance à au moins 92% de résultats positifs vrais dans tous les cas du test 4, comme au moins 95%, 97%, 98%, 99%, 99,5%, et 99,9% de résultats positifs vrais.

38. Procédé selon l'une quelconque des revendications 23-37, dans lequel la détermination donne naissance à au plus 20% de résultats positifs faux dans le test 5, comme au plus 15%, 10%, 6%,3%, 2%, 1%, 1/2%.

39. Procédé selon l'une quelconque des revendications précédentes, dans lequel l'échantillon est pris chez un animal, y compris un être humain.

40. Procédé selon la revendication 39, dans lequel l'origine de l'échantillon est choisie dans le groupe constitué par les fèces, le suc gastrique, l'urine, le sang, la lymphe, le liquide cérébrospinal et une biopsie.

41. Procédé selon l'une quelconque des revendications 1-38, dans lequel l'échantillon provient de l'environnement.

42. Procédé selon la revendication 41, dans lequel l'origine de l'échantillon est choisie dans le groupe constitué par la poussière, les tampons, et le duvet.

43. Procédé selon l'une quelconque des revendications 23-38, dans lequel l'échantillon est un produit traité.

44. Procédé selon l'une quelconque des revendications 1-38 et 43, dans lequel l'échantillon est pris dans un aliment ou un fourrage.

45. Procédé selon l'une quelconque des revendications précédentes, dans lequel l'étape de détermination comprend une réaction immunologique.

46. Procédé selon la revendication 45, dans lequel l'étape de détermination comprend un essai choisi dans le groupe constitué par un essai immunologique, comme un essai radio-immunologique (RIA), un essai immunoenzymatique (EIA), un essai d'immunosorption liée à l'enzyme (ELISA), une technique d'anticorps par fluorescence (FA), et un essai immun comprenant l'utilisation de liposomes, de micelles ou de particules de type liposome dans la phase de détection;
un essai faisant intervenir une immobilisation immune ;
un essai faisant intervenir une agglutination immune comme l'agglutination du latex ;
un essai d'hybridation ADN/ADN ;
un essai d'hybridation ARN/ARN ; et
un essai d'hybridation ADN/ARN ;

47. Procédé selon l'une quelconque des revendications 1-44, dans lequel l'étape de détermination comprend une amplification en chaîne par polymérase (PCR).

48. Procédé selon l'une quelconque des revendications 1-44, dans lequel l'étape de détermination comprend l'utilisation d'acides nucléiques de peptides (PNA).

49. Procédé selon l'une quelconque des revendications 1-44, dans lequel l'étape de détermination comprend l'utilisation de lectines.

50. Procédé selon l'une quelconque des revendications 1-44, dans lequel l'étape de détermination comprend l'utilisation de bactériophages, comme le bactériophage Felix 01.

51. Procédé selon l'une quelconque des revendications 1-44, dans lequel l'étape de détermination comprend une conversion métabolique par la *Salmonella* d'une substance qui aboutit à une modification de la résistance électrique ou de l'impédance du milieu contenant la *Salmonella* et dans lequel la détection de la *Salmonella* se fait par mesure de ce changement.

52. Procédé selon l'une quelconque des revendications 1-44, dans lequel l'étape de détermination comprend un essai de l'aptitude de *Salmonella* à migrer sur ou à travers une matrice de gélose.

53. Procédé selon l'une quelconque des revendications 1-44, dans lequel l'étape de détermination comprend un essai faisant intervenir une étape de filtration, dans laquelle les bactéries sont séparées du milieu et sont retenues sur un filtre, comme un essai faisant intervenir un procédé à filtre de membrane à grille hydrophobe (HGMF).

54. Procédé selon l'une quelconque des revendications 1-44, dans lequel l'étape de détermination comprend un essai faisant intervenir un procédé radiométrique.

55. Procédé selon l'une quelconque des revendications 1-44, dans lequel l'étape de détermination comprend un essai faisant intervenir une séparation chromatographique.

56. Procédé selon l'une quelconque des revendications précédentes, dans lequel l'étape de détermination fait intervenir un post-enrichissement sélectif.

57. Procédé selon la revendication 56, dans lequel le post-enrichissement sélectif a une durée de 2-6 heures, comme une durée de 2,0-4,5 heures, de 2,5-3,5 heures, et de préférence d'environ 3 heures.

58. Procédé selon l'une quelconque des revendications précédentes, dans lequel le milieu de croissance aqueux pour *Salmonella* a une concentration de sélénite correspondant au plus à la concentration de sélénite dans une solution de sélénite acide de sodium à 0,5% p/v, de préférence une concentration en sélénite de 0% p/v.
